# EUROPEAN PATENT SPECIFICATION

(11) **EP 1 804 651 B1**
(45) Date of publication and mention of the grant of the patent: **30.03.2016**
(21) Application number: 05796498.3
(22) Date of filing: 15.09.2005
(51) Int. Cl.: A61B 5/00, B65D 81/00

(54) **APPARATUS FOR AN IMPROVED SAMPLE CAPTURE**
VERBESSERTES PROBENNAHMEGERÄT
DISPOSITIF DE CAPTURE D'ECHANTILLONS AMELIORE

(30) Priority: 15.09.2004 US 610305 P; 15.09.2004 US 610360 P; 16.09.2004 US 611094 P
(43) Date of publication of application: 11.07.2007
(73) Proprietor: Sanofi-Aventis Deutschland GmbH, 65929 Frankfurt am Main (DE)
(72) Inventor: DESHMUKH, Ajay, Mountain View, CA 94041 (US); MARSOT, Travis, Mountain View, CA 94043 (US); SCHUMANN, Matt, Cambridge CB3 7RY (GB); ALDEN, Don, Sunnyvale, CA 94087 (US)
(74) Representative: McDougall, James
(86) International application number: PCT/US2005/032810
(87) International publication number: WO 2006/031920

(56) References cited:
- WO-A-2005/120199
- WO-A2-2005/016125
- WO-A2-2005/033659
- US-A1- 2003 199 893
- US-B2- 6 540 675
- US-B2- 6 591 124

## Description

### BACKGROUND OF THE INVENTION

### Technical Field:

The technical field relates to analyte detecting devices, and more specifically, coatings for improving glucose measurement.

### Background Art:

Lancing devices are known in the medical health-care products industry for piercing the skin to produce blood for analysis. Typically, a drop of blood for this type of analysis is obtained by making a small incision in the fingertip, creating a small wound, which generates a small blood droplet on the surface of the skin.

Early methods of lancing included piercing or slicing the skin with a needle or razor. An example of a piercing device is described in US 2003/0199893. Current methods utilize lancing devices that contain a multitude of spring, cam and mass actuators to drive the lancet. These include cantilever springs, diaphragms, coil springs, as well as gravity plumbs used to drive the lancet. The device may be held against the skin and mechanically triggered to ballistically launch the lancet. Unfortunately, the pain associated with each lancing event using known technology discourages patients from testing. In addition to vibratory stimulation of the skin as the driver impacts the end of a launcher stop, known spring based devices have the possibility of firing lancets that harmonically oscillate against the patient tissue, causing multiple strikes due to recoil. This recoil and multiple strikes of the lancet is one major impediment to patient compliance with a structured glucose monitoring regime.

Success rate generally encompasses the probability of producing a blood sample with one lancing action, which is sufficient in volume to perform the desired analytical test. The blood may appear spontaneously at the surface of the skin, or may be "milked" from the wound. Milking generally involves pressing the side of the digit, or in proximity of the wound to express the blood to the surface. In traditional methods, the blood droplet produced by the lancing action must reach the surface of the skin to be viable for testing.

When using existing methods, blood often flows from the cut blood vessels but is then trapped below the surface of the skin, forming a hematoma. In other instances, a wound is created, but no blood flows from the wound. In either case, the lancing process cannot be combined with the sample acquisition and testing step. Spontaneous blood droplet generation with current mechanical launching system varies between launcher types but on average it is about 50% of lancet strikes, which would be spontaneous. Otherwise milking is required to yield blood. Mechanical launchers are unlikely to provide the means for integrated sample acquisition and testing if one out of every two strikes does not yield a spontaneous blood sample.

Many diabetic patients (insulin dependent) are required to self-test for blood glucose levels five to six times daily. The large number of steps required in traditional methods of glucose testing ranging from lancing, to milking of blood, applying blood to the test strip, and getting the measurements from the test strip discourages many diabetic patients from testing their blood glucose levels as often as recommended. Tight control of plasma glucose through frequent testing is therefore mandatory for disease management. The pain associated with each lancing event further discourages patients from testing. Additionally, the wound channel left on the patient by known systems may also be of a size that discourages those who are active with their hands or who are worried about healing of those wound channels from testing their glucose levels.

Another problem frequently encountered by patients who must use lancing equipment to obtain and analyze blood samples is the amount of manual dexterity and hand-eye coordination required to properly operate the lancing and sample testing equipment due to retinopathies and neuropathies particularly, severe in elderly diabetic patients. For those patients, operating existing lancet and sample testing equipment can be a challenge. Once a blood droplet is created, that droplet must then be guided into a receiving channel of a small test strip or the like. If the sample placement on the strip is unsuccessful, repetition of the entire procedure including relancing the skin to obtain a new blood droplet is desired.

Early methods of using test strips required a relatively substantial volume of blood to obtain an accurate glucose measurement. This large blood requirement made the monitoring experience a painful one for the user since the user may need to lance deeper than comfortable to obtain sufficient blood generation. Alternatively, if insufficient blood is spontaneously generated, the user may need to "milk" the wound to squeeze enough blood to the skin surface. Neither method is desirable as they take additional user effort and may be painful. The discomfort and inconvenience associated with such lancing events may deter a user from testing their blood glucose levels in a rigorous manner sufficient to control their diabetes.

A further impediment to patient compliance is the amount of time that at lower volumes, it becomes even more important that blood or other fluid sample be directed to a measurement device without being wasted or spilled along the way. Known devices do not effectively handle the low sample volumes in an efficient manner. Accordingly, improved sensing devices are desired to increase user compliance and reduce the hurdles associated with analyte measurement.

WO/2005/120199 (state of the art according to Article 54(3) EPC) discloses an analyte detecting device. The device comprises a cartridge having a plurality of cavities and a plurality of penetrating members at least partially contained in the cavities of the single cartridge wherein the penetrating members are slidably movable to extend outward from lateral openings on the cartridge to penetrate tissue. The device may also include a sterility barrier coupled to the cartridge, the sterility barrier covering a plurality of the lateral openings, wherein the sterility barrier covering the lateral openings is configured to be moved so that a penetrating member exits the lateral opening without contacting the barrier; a plurality of analyte detecting members coupled to an underside of the cartridge and a plurality of sample capture devices, the sample capture devices each having a vertical portion and a horizontal portion, the vertical portion having a opening there through to allow a penetrating member to pass through.

WO/2005/033659 (state of the art according to Article 54(3) EPC) discloses a body fluid sampling device. In the device a mesh may be used to guide blood or fluid to travel directly form the wound to an analyte detecting port on the cartridge. Thus the volume of blood or body fluid produced at the wound site regardless of its droplet geometry can be reliable and substantially completely transported to the analyte detecting member for measurement.

WO/2005/016125 (state of the art according to Article 54(3) EPC) discloses a body fluid sampling device. The device includes a cartridge containing a plurality of penetrating members and a second portion having a plurality of analyte detecting members and where the second portion is coupled to the first cartridge. The analyte detecting members on the second portion are positioned to receive body fluid for a wound created by one of the penetrating members exiting the first cartridge.

### SUMMARY OF THE INVENTION

Aspects of the invention are set out in the appended claims.

The present invention which is defined in claim 1 provides solutions for at least some of the drawbacks discussed above. Specifically, some embodiments of the present invention provide an improved apparatus for measuring analyte levels in a body fluid. The present disclosure also provided improved techniques for manufacturing such analyte detecting devices. A device is provided comprising a cartridge having a plurality of cavities. The device may include a plurality of penetrating members at least partially contained in the cavities of the single cartridge wherein the penetrating members are slidably movable to extend outward from lateral openings on the cartridge to penetrate tissue. The device may have a sterility barrier coupled to the cartridge, wherein the sterility barrier covers a plurality of the lateral openings, and wherein the sterility barrier covering the lateral openings is configured to be moved so that a penetrating member exits the lateral opening without contacting the barrier. The device may include a plurality of analyte detecting members coupled to the cartridge and a plurality of sample capture devices, wherein the sample capture devices each having an opening there through to allow a penetrating member to pass through.

The sample capture devices may be L shaped. The sample capture devices may be formed on a ribbon or tape structure. The sample capture devices may be formed on circular disc and coupled to the cartridge. The sample capture devices may each include a wicking member. The sample capture devices may each include a hydrophilic membrane. The sample capture devices may each include a wicking member and a capillary member. The sample capture devices may each have a lollipop shaped opening on the vertical portion. The sample capture devices may each be individually movable to be in a valve open or a valve closed position. The sample capture devices may each be hinged to a disc.

A method of manufacturing an analyte detecting device comprises providing a housing and providing a cartridge that is sized to fit within the housing. An opening may be formed on the housing. At least one layer of viscoelastic material may be applied on the housing around the opening, wherein the material applying an compression force to a target tissue when the target tissue engages said material. The method may also include providing a plurality of penetrating members in said cartridge and providing a plurality of analyte detection devices in said cartridge.

The method may include using analyte detection devices to measure glucose levels in a sample fluid. The penetrating members may be lancets. The method may include placing a solenoid penetrating member driver in the housing. The viscoelastic material may be formed in the shape of an O-ring. The viscoelastic material may be formed to have a primary portion and a secondary portion, wherein the secondary portion engages a portion of the tissue away from a lancing site and compressing the tissue to drive blood towards the lancing site. The method may involve providing a plurality of seals on the cartridge to maintain the penetrating members in a sterile condition prior to use. An LCD screen may be provided on the housing to provide information to the user during use. An LCD screen may be provided on the housing to provide information to the user during use.

At least some of these and other objectives described herein will be met by embodiments of the present invention.

### BRIEF DESCRIPTION OF THE DRAWINGS

Figure 1 illustrates an embodiment of a controllable force driver in the form of a cylindrical electric penetrating member driver using a coiled solenoid -type configuration.
Figure 2A illustrates a displacement over time profile of a penetrating member driven by a harmonic spring/mass system.
Figure 2B illustrates the velocity over time profile of a penetrating member driver by a harmonic spring/mass system.
Figure 2C illustrates a displacement over time profile of an embodiment of a controllable force driver.
Figure 2D illustrates a velocity over time profile of an embodiment of a controllable force driver.
Figure 3 is a diagrammatic view illustrating a controlled feed-back loop.
Figure 4 is a perspective view of a tissue penetration device having features of the invention.
Figure 5 is an elevation view in partial longitudinal section of the tissue penetration device of Figure 4.
Figure 6 shows an exploded perspective view of one embodiment of a device according to the present invention.
Figure 7 shows an exploded perspective view of a penetrating member cartridge.
Figure 8 shows one embodiment of a sensing layer.
Figure 9 shows one embodiment of a testing device.
Figure 10 shows a ribbon capable of multiple testing events.
Figure 11 shows an exploded perspective view of one embodiment.
Figure 12 and 13 show cutaway views of the embodiments of the present invention.
Figure 14 and 5 show one embodiment of the present invention.
Figures 16 and 17 show another embodiment according to the present invention.
Figure 18 through 20 show various view of a device based on a disc.
Figures 21 through 23 shows various view of a device based on individual testing devices.
Figures 24 through 27 show various view of a device based on a ribbon.
Figures 28 through 31 show embodiments using foils and adhesives.
Figures 32 and 33 show a device with an airtight seal with the outer ring and the inner ring.
Figures 34 and 35 show other embodiments using adhesives according to the present invention.
Figures 36 and 37 show an embodiment where a cover film is removed to unseal analyte detecting members.
Figures 38 though 41 show an embodiment where a cover film is removed to unseal analyte detecting members.
Figures 42 though 46 show an embodiment where a cover film is removed to unseal analyte detecting members.
Figures 47A through 47C show exploded perspective views of various devices according to the present invention.
Figures 48A through 48C show exploded perspective views of various devices according to the present invention.
Figures 49A through 49B show exploded perspective views of various devices according to the present invention.
Figure 50 shows a close-up view of one embodiment of a ribbon according to the present invention.
Figure 51 through 54 show various views of another embodiment of the present invention.
Figures 55 and 56 show various views of one embodiment of the present invention.
Figure 57 shows a dissolvable film device.
Figure 58 and 59 show embodiments of front ends according to the present invention.
Figures 60 and 61 shows another view of a device according to the present invention.
Figure 61 shows one method of connection according to the present invention.
Figures 63 and 64 show a dissolvable concept.
Figure 65 shows a fluid pathway.
Figures 66 through 68 show front ends according to the present invention.
Figure 69 shows a front end on an integrated lancing device with an LCD or user display.
Figures 70 through 71 show another embodiment of the present invention.
Figures 72 through 73 show electrode positioning.
Figure 74 shows an embodiment where the analyte detecting members may be in motion.
Figure 75 shows a perspective view of another embodiment according to the present invention.
Figures 76 and 77 show various view of devices according to the present invention.
Figure 78 through 81 other embodiments according to the present invention using various cartridges.

### DESCRIPTION OF THE SPECIFIC EMBODIMENTS

It is to be understood that both the foregoing general description and the following detailed description are exemplary and explanatory only and are not restrictive of the invention, as claimed. It may be noted that, as used in the specification and the appended claims, the singular forms "a", "an" and "the" include plural referents unless the context clearly dictates otherwise. Thus, for example, reference to "a material" may include mixtures of materials, reference to "a chamber" may include multiple chambers, and the like.

In this specification and in the claims which follow, reference will be made to a number of terms which shall be defined to have the following meanings:
"Optional" or "optionally" means that the subsequently described circumstance may or may not occur, so that the description includes instances where the circumstance occurs and instances where it does not. For example, if a device optionally contains a feature for analyzing a blood sample, this means that the analysis feature may or may not be present, and, thus, the description includes structures wherein a device possesses the analysis feature and structures wherein the analysis feature is not present.

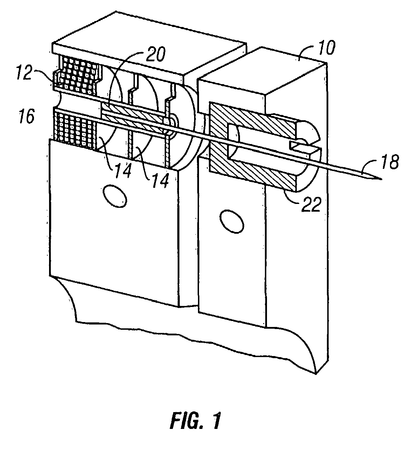

The present invention may be used with a variety of different penetrating member drivers. It is contemplated that these penetrating member drivers may be spring based, solenoid based, magnetic driver based, nanomuscle based, or based on any other mechanism useful in moving a penetrating member along a path into tissue. It should be noted that the present invention is not limited by the type of driver used with the penetrating member feed mechanism. One suitable penetrating member driver for use with the present invention is shown in Figure 1. This is an embodiment of a solenoid type electromagnetic driver that is capable of driving an iron core or slug mounted to the penetrating member assembly using a direct current (DC) power supply. The electromagnetic driver includes a driver coil pack that is divided into three separate coils along the path of the penetrating member, two end coils and a middle coil. Direct current is alternated to the coils to advance and retract the penetrating member. Although the driver coil pack is shown with three coils, any suitable number of coils may be used, for example, 4, 5, 6, 7 or more coils may be used.

Referring to the embodiment of Figure 1, the stationary iron housing 10 may contain the driver coil pack with a first coil 12 flanked by iron spacers 14 which concentrate the magnetic flux at the inner diameter creating magnetic poles. The inner insulating housing 16 isolates the penetrating member 18 and iron core 20 from the coils and provides a smooth, low friction guide surface. The penetrating member guide 22 further centers the penetrating member 18 and iron core 20. The penetrating member 18 is protracted and retracted by alternating the current between the first coil 12, the middle coil, and the third coil to attract the iron core 20. Reversing the coil sequence and attracting the core and penetrating member back into the housing retracts the penetrating member. The penetrating member guide 22 also serves as a stop for the iron core 20 mounted to the penetrating member 18.

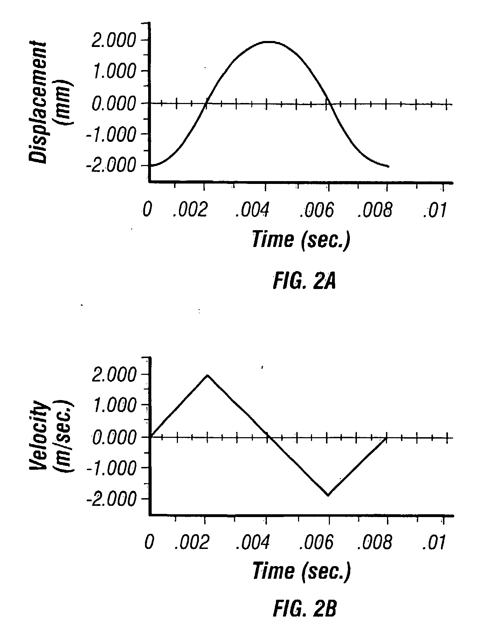

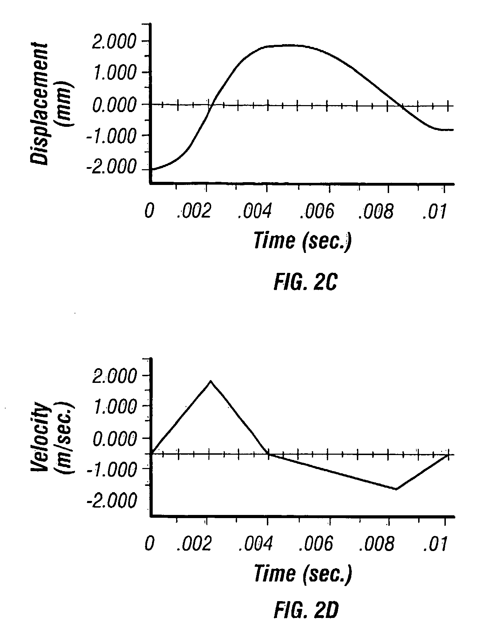

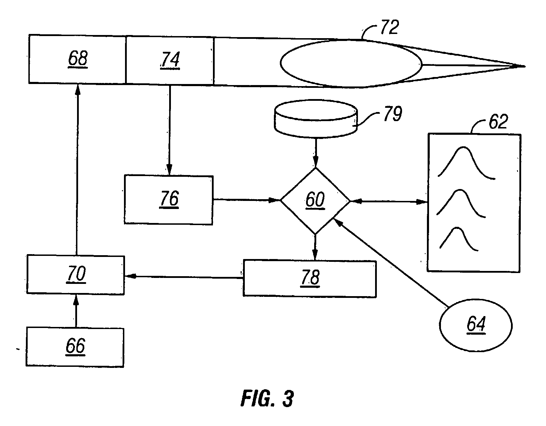

As discussed above, tissue penetration devices which employ spring or cam driving methods have a symmetrical or nearly symmetrical actuation displacement and velocity profiles on the advancement and retraction of the penetrating member as shown in Figures 2 and 3. In most of the available lancet devices, once the launch is initiated, the stored energy determines the velocity profile until the energy is dissipated. Controlling impact, retraction velocity, and dwell time of the penetrating member within the tissue can be useful in order to achieve a high success rate while accommodating variations in skin properties and minimize pain. Advantages can be achieved by taking into account of the fact that tissue dwell time is related to the amount of skin deformation as the penetrating member tries to puncture the surface of the skin and variance in skin deformation from patient to patient based on skin hydration.

In this embodiment, the ability to control velocity and depth of penetration may be achieved by use of a controllable force driver where feedback is an integral part of driver control. Such drivers can control either metal or polymeric penetrating members or any other type of tissue penetration element. The dynamic control of such a driver is illustrated in Figure. 2C which illustrates an embodiment of a controlled displacement profile and Figure 2D which illustrates an embodiment of a the controlled velocity profile. These are compared to Figures 2A and 2B, which illustrate embodiments of displacement and velocity profiles, respectively, of a harmonic spring/mass powered driver. Reduced pain can be achieved by using impact velocities of greater than about 2 m/s entry of a tissue penetrating element, such as a lancet, into tissue. Other suitable embodiments of the penetrating member driver are described in commonly assigned, copending U.S. Patent Application Ser. No. 10/127,395, (Attorney Docket No. 38187-2551) filed April 19, 2002 and previously incorporated herein.

Figure 3 illustrates the operation of a feedback loop using a processor 60. The processor 60 stores profiles 62 in non-volatile memory. A user inputs information 64 about the desired circumstances or parameters for a lancing event. The processor 60 selects a driver profile 62 from a set of alternative driver profiles that have been preprogrammed in the processor 60 based on typical or desired tissue penetration device performance determined through testing at the factory or as programmed in by the operator. The processor 60 may customize by either scaling or modifying the profile based on additional user input information 64. Once the processor has chosen and customized the profile, the processor 60 is ready to modulate the power from the ower supply 66 to the penetrating member driver 68 through an amplifier 70. The processor 60 may measure the location of the penetrating member 72 using a position sensing mechanism 74 through an analog to digital converter 76 linear encoder or other such transducer. Examples of position sensing mechanisms have been described in the embodiments above and may be found in the specification for commonly assigned, copending U.S. Patent Application Ser. No. 10/127,395, (Attorney Docket No. 38187-2551) filed April 19, 2002 and previously incorporated herein. The processor 60 calculates the movement of the penetrating member by comparing the actual profile of the penetrating member to the predetermined profile. The processor 60 modulates the power to the penetrating member driver 68 through a signal generator 78, which may control the amplifier 70 so that the actual velocity profile of the penetrating member does not exceed the predetermined profile by more than a preset error limit. The error limit is the accuracy in the control of the penetrating member.

After the lancing event, the processor 60 can allow the user to rank the results of the lancing event. The processor 60 stores these results and constructs a database 80 for the individual user. Using the database 79, the processor 60 calculates the profile traits such as degree of painlessness, success rate, and blood volume for various profiles 62 depending on user input information 64 to optimize the profile to the individual user for subsequent lancing cycles. These profile traits depend on the characteristic phases of penetrating member advancement and retraction. The processor 60 uses these calculations to optimize profiles 62 for each user. In addition to user input information 64, an internal clock allows storage in the database 79 of information such as the time of day to generate a time stamp for the lancing event and the time between lancing events to anticipate the user's diurnal needs. The database stores information and statistics for each user and each profile that particular user uses.

In addition to varying the profiles, the processor 60 can be used to calculate the appropriate penetrating member diameter and geometry suitable to realize the blood volume required by the user. For example, if the user requires about 1-5 microliter volume of blood, the processor 60 may select a 200 micron diameter penetrating member to achieve these results. For each class of penetrating member, both diameter and penetrating member tip geometry, is stored in the processor 60 to correspond with upper and lower limits of attainable blood volume based on the predetermined displacement and velocity profiles.

The lancing device is capable of prompting the user for information at the beginning and the end of the lancing event to more adequately suit the user. The goal is to either change to a different profile or modify an existing profile. Once the profile is set, the force driving the penetrating member is varied during advancement and retraction to follow the profile. The method of lancing using the lancing device comprises selecting a profile, lancing according to the selected profile, determining lancing profile traits for each characteristic phase of the lancing cycle, and optimizing profile traits for subsequent lancing events.

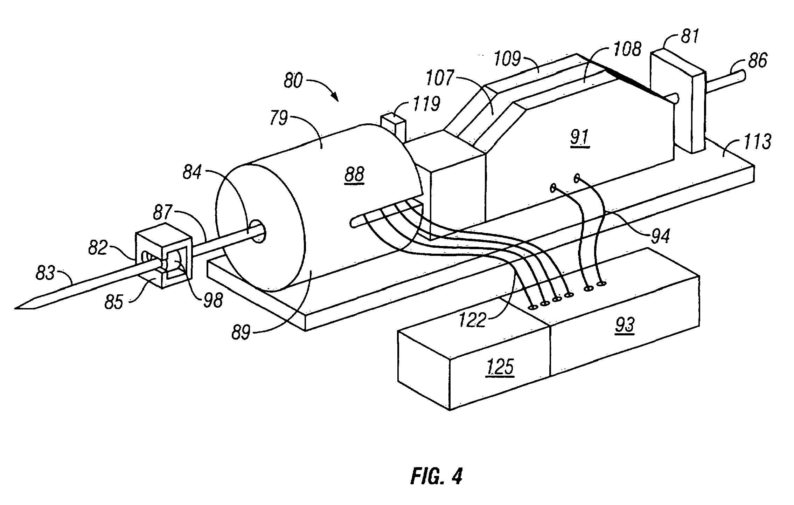

Figure 4 illustrates an embodiment of a tissue penetration device, more specifically, a lancing device 80 that includes a controllable driver 179 coupled to a tissue penetration element. The lancing device 80 has a proximal end 81 and a distal end 82. At the distal end 82 is the tissue penetration element in the form of a penetrating member 83, which is coupled to an elongate coupler shaft 84 by a drive coupler 85. The elongate coupler shaft 84 has a proximal end 86 and a distal end 87. A driver coil pack 88 is disposed about the elongate coupler shaft 84 proximal of the penetrating member 83. A position sensor 91 is disposed about a proximal portion 92 of the elongate coupler shaft 84 and an electrical conductor 94 electrically couples a processor 93 to the position sensor 91. The elongate coupler shaft 84 driven by the driver coil pack 88 controlled by the position sensor 91 and processor 93 form the controllable driver, specifically, a controllable electromagnetic driver.

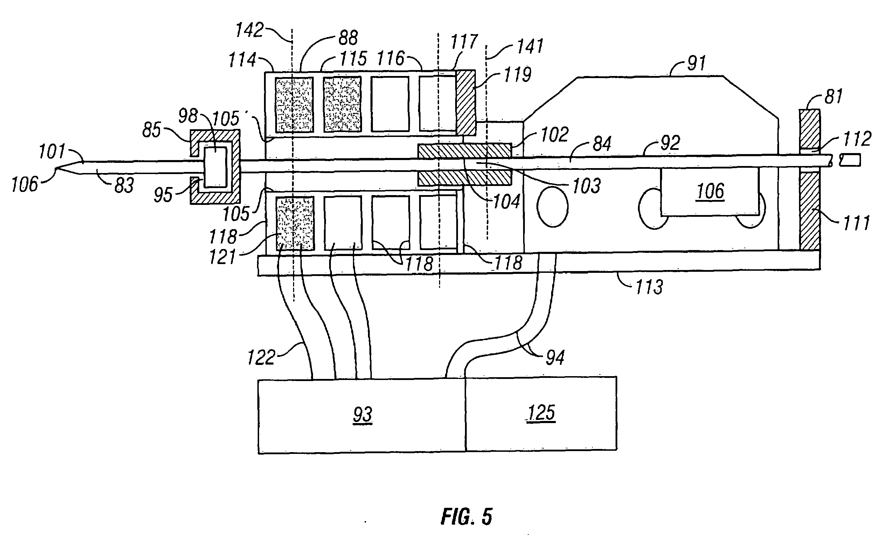

Referring to Figure 5, the lancing device 80 can be seen in more detail, in partial longitudinal section. The penetrating member 83 has a proximal end 95 and a distal end 96 with a sharpened point at the distal end 96 of the penetrating member 83 and a drive head 98 disposed at the proximal end 95 of the penetrating member 83. A penetrating member shaft 201 is disposed between the drive head 98 and the sharpened point 97. The penetrating member shaft 201 may be comprised of stainless steel, or any other suitable material or alloy and have a transverse dimension of about 0.1 to about 0.4 mm. The penetrating member shaft may have a length of about 3 mm to about 50 mm, specifically, about 15 mm to about 20 mm. The drive head 98 of the penetrating member 83 is an enlarged portion having a transverse dimension greater than a transverse dimension of the penetrating member shaft 201 distal of the drive head 98. This configuration allows the drive head 98 to be mechanically captured by the drive coupler 85. The drive head 98 may have a transverse dimension of about 0.5 to about 2 mm.

A magnetic member 102 is secured to the elongate coupler shaft 84 proximal of the drive coupler 85 on a distal portion 203 of the elongate coupler shaft 84. The magnetic member 102 is a substantially cylindrical piece of magnetic material having an axial lumen 204 extending the length of the magnetic member 102. The magnetic member 102 has an outer transverse dimension that allows the magnetic member 102 to slide easily within an axial lumen 105 of a low friction, possibly lubricious, polymer guide tube 105' disposed within the driver coil pack 88. The magnetic member 102 may have an outer transverse dimension of about 1.0 to about 5.0 mm, specifically, about 2.3 to about 2.5 mm. The magnetic member 102 may have a length of about 3.0 to about 5.0 mm, specifically, about 4.7 to about 4.9 mm. The magnetic member 102 can be made from a variety of magnetic materials including ferrous metals such as ferrous steel, iron, ferrite, or the like. The magnetic member 102 may be secured to the distal portion 203 of the elongate coupler shaft 84 by a variety of methods including adhesive or epoxy bonding, welding, crimping or any other suitable method.

Proximal of the magnetic member 102, an optical encoder flag 206 is secured to the elongate coupler shaft 84. The optical encoder flag 206 is configured to move within a slot 107 in the position sensor 91. The slot 107 of the position sensor 91 is formed between a first body portion 108 and a second body portion 109 of the position sensor 91. The slot 107 may have separation width of about 1.5 to about 2.0 mm. The optical encoder flag 206 can have a length of about 14 to about 18 mm, a width of about 3 to about 5 mm and a thickness of about 0.04 to about 0.06 mm.

The optical encoder flag 206 interacts with various optical beams generated by LEDs disposed on or in the position sensor body portions 108 and 109 in a predetermined manner. The interaction of the optical beams generated by the LEDs of the position sensor 91 generates a signal that indicates the longitudinal position of the optical flag 206 relative to the position sensor 91 with a substantially high degree of resolution. The resolution of the position sensor 91 may be about 200 to about 400 cycles per inch, specifically, about 350 to about 370 cycles per inch. The position sensor 91 may have a speed response time (position/time resolution) of 0 to about 120,000 Hz, where one dark and light stripe of the flag constitutes one Hertz, or cycle per second. The position of the optical encoder flag 206 relative to the magnetic member 102, driver coil pack 88 and position sensor 91 is such that the optical encoder 91 can provide precise positional information about the penetrating member 83 over the entire length of the penetrating member's power stroke.

An optical encoder that is suitable for the position sensor 91 is a linear optical incremental encoder, model HEDS 9200, manufactured by Agilent Technologies. The model HEDS 9200 may have a length of about 20 to about 30 mm, a width of about 8 to about 12 mm, and a height of about 9 to about 11 mm. Although the position sensor 91 illustrated is a linear optical incremental encoder, other suitable position sensor embodiments could be used, provided they posses the requisite positional resolution and time response. The HEDS 9200 is a two channel device where the channels are 90 degrees out of phase with each other. This results in a resolution of four times the basic cycle of the flag. These quadrature outputs make it possible for the processor to determine the direction of penetrating member travel. Other suitable position sensors include capacitive encoders, analog reflective sensors, such as the reflective position sensor discussed above, and the like.

A coupler shaft guide 111 is disposed towards the proximal end 81 of the lancing device 80. The guide 111 has a guide lumen 112 disposed in the guide 111 to slidingly accept the proximal portion 92 of the elongate coupler shaft 84. The guide 111 keeps the elongate coupler shaft 84 centered horizontally and vertically in the slot 102 of the optical encoder 91.

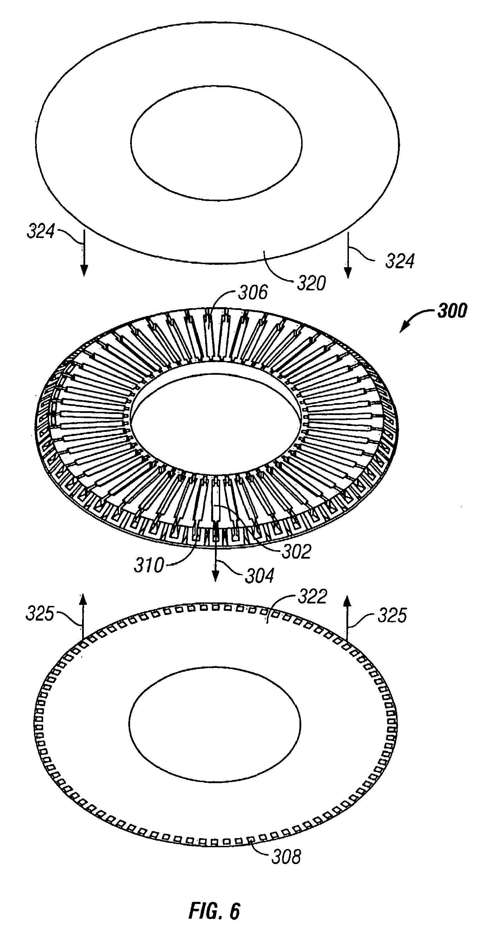

Referring now to Figure 6, a still further embodiment of a cartridge according to the present invention will be described. Figure 6 shows one embodiment of a cartridge 300 which may be removably inserted into an apparatus for driving penetrating members to pierce skin or tissue. The cartridge 300 has a plurality of penetrating members 302 that may be individually or otherwise selectively actuated so that the penetrating members 302 may extend outward from the cartridge, as indicated by arrow 304, to penetrate tissue. In the present embodiment, the cartridge 300 may be based on a flat disc with a number of penetrating members such as, but in no way limited to, (25, 50, 75, 100, ...) arranged radially on the disc or cartridge 800. It should be understood that although the cartridge 300 is shown as a disc or a disc-shaped housing, other shapes or configurations of the cartridge may also work without departing from the spirit of the present invention of placing a plurality of penetrating members to be engaged, singly or in some combination, by a penetrating member driver.

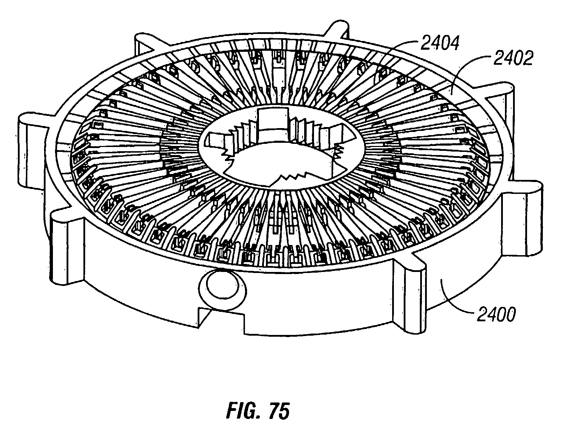

Each penetrating member 302 may be contained in a cavity 306 in the cartridge 300 with the penetrating member's sharpened end facing radially outward and may be in the same plane as that of the cartridge. The cavity 306 may be molded, pressed, forged, or otherwise formed in the cartridge. Although not limited in this manner, the ends of the cavities 306 may be divided into individual fingers (such as one for each cavity) on the outer periphery of the disc. The particular shape of each cavity 306 may be designed to suit the size or shape of the penetrating member therein or the amount of space desired for placement of the analyte detecting members 808. For example and not limitation, the cavity 306 may have a V-shaped cross-section, a U-shaped cross-section, C-shaped cross-section, a multi-level cross section or the other cross-sections. The opening 810 through which a penetrating member 302 may exit to penetrate tissue may also have a variety of shapes, such as but not limited to, a circular opening, a square or rectangular opening, a U-shaped opening, a narrow opening that only allows the penetrating member to pass, an opening with more clearance on the sides, a slit, a configuration as shown in Figure 75, or the other shapes.

In this embodiment, after actuation, the penetrating member 302 is returned into the cartridge and may be held within the cartridge 300 in a manner so that it is not able to be used again. By way of example and not limitation, a used penetrating member may be returned into the cartridge and held by the launcher in position until the next lancing event. At the time of the next lancing, the launcher may disengage the used penetrating member with the cartridge 300 turned or indexed to the next clean penetrating member such that the cavity holding the used penetrating member is position so that it is not accessible to the user (i.e. turn away from a penetrating member exit opening). In some embodiments, the tip of a used penetrating member may be driven into a protective stop that hold the penetrating member in place after use. The cartridge 300 is replaceable with a new cartridge 300 once all the penetrating members have been used or at such other time or condition as deemed desirable by the user.

Referring still to the embodiment in Figure 6, the cartridge 300 may provide sterile environments for penetrating members via seals, foils, covers, polymeric, or similar materials used to seal the cavities and provide enclosed areas for the penetrating members to rest in. In the present embodiment, a foil or seal layer 320 is applied to one surface of the cartridge 300. The seal layer 320 may be made of a variety of materials such as a metallic foil or other seal materials and may be of a tensile strength and other quality that may provide a sealed, sterile environment until the seal layer 320 is penetrate by a suitable or penetrating device providing a preselected or selected amount of force to open the sealed, sterile environment. Each cavity 306 may be individually sealed with a layer 320 in a manner such that the opening of one cavity does not interfere with the sterility in an adjacent or other cavity in the cartridge 800. As seen in the embodiment of Figure 6, the seal layer 320 may be a planar material that is adhered to a top surface of the cartridge 800.

Depending on the orientation of the cartridge 300 in the penetrating member driver apparatus, the seal layer 320 may be on the top surface, side surface, bottom surface, or other positioned surface. For ease of illustration and discussion of the embodiment of Figure 6, the layer 320 is placed on a top surface of the cartridge 800. The cavities 306 holding the penetrating members 302 are sealed on by the foil layer 320 and thus create the sterile environments for the penetrating members. The foil layer 320 may seal a plurality of cavities 306 or only a select number of cavities as desired.

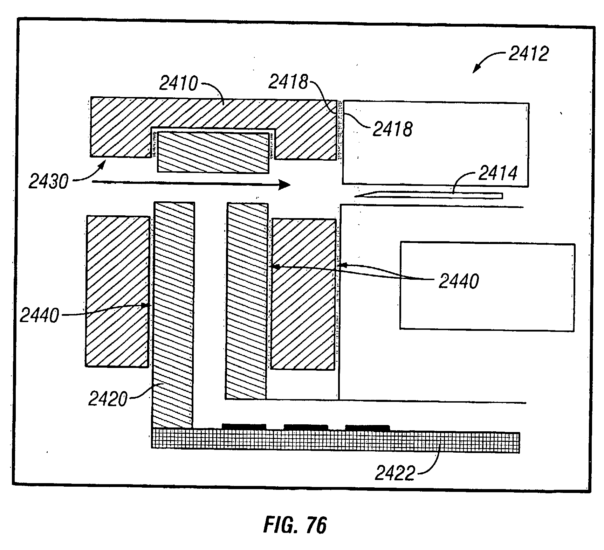

In a still further feature of Figure 6, the cartridge 300 may optionally include a plurality of analyte detecting members 308 on a substrate 822 which may be attached to a bottom surface of the cartridge 300. The substrate may be made of a material such as, but not limited to, a polymer, a foil, or other material suitable for attaching to a cartridge and holding the analyte detecting members 308. As seen in Figure 6, the substrate 322 may hold a plurality of analyte detecting members, such as but not limited to, about 10-50, 50-100, or other combinations of analyte detecting members. This facilitates the assembly and integration of analyte detecting members 308 with cartridge 300. These analyte detecting members 308 may enable an integrated body fluid sampling system where the penetrating members 302 create a wound tract in a target tissue, which expresses body fluid that flows into the cartridge for analyte detection by at least one of the analyte detecting members 308. The substrate 322 may contain any number of analyte detecting members 308 suitable for detecting analytes in cartridge having a plurality of cavities 306. In one embodiment, many analyte detecting members 308 may be printed onto a single substrate 322 which is then adhered to the cartridge to facilitate manufacturing and simplify assembly. The analyte detecting members 308 may be electrochemical in nature. The analyte detecting members 308 may further contain enzymes, dyes, or other detectors which react when exposed to the desired analyte. Additionally, the analyte detecting members 308 may comprise of clear optical windows that allow light to pass into the body fluid for analyte analysis. The number, location, and type of analyte detecting member 308 may be varied as desired, based in part on the design of the cartridge, number of analytes to be measured, the need for analyte detecting member calibration, and the sensitivity of the analyte detecting members. If the cartridge 300 uses an analyte detecting member arrangement where the analyte detecting members are on a substrate attached to the bottom of the cartridge, there may be through holes (as shown in Figure 76), wicking elements, capillary tube or other devices on the cartridge 300 to allow body fluid to flow from the cartridge to the analyte detecting members 308 for analysis. In other configurations, the analyte detecting members 308 may be printed, formed, or otherwise located directly in the cavities housing the penetrating members 302 or areas on the cartridge surface that receive blood after lancing.

The use of the seal layer 320 and substrate or analyte detecting member layer 822 may facilitate the manufacture of these cartridges 10. For example, a single seal layer 320 may be adhered, attached, or otherwise coupled to the cartridge 300 as indicated by arrows 324 to seal many of the cavities 306 at one time. A sheet 322 of analyte detecting members may also be adhered, attached, or otherwise coupled to the cartridge 300 as indicated by arrows 325 to provide many analyte detecting members on the cartridge at one time. During manufacturing of one embodiment of the present invention, the cartridge 300 may be loaded with penetrating members 302, sealed with layer 320 and a temporary layer (not shown) on the bottom where substrate 322 would later go, to provide a sealed environment for the penetrating members. This assembly with the temporary bottom layer is then taken to be sterilized. After sterilization, the assembly is taken to a clean room (or it may already be in a clear room or equivalent environment) where the temporary bottom layer is removed and the substrate 322 with analyte detecting members is coupled to the cartridge as shown in Figure 6. This process allows for the sterile assembly of the cartridge with the penetrating members 302 using processes and/or temperatures that may degrade the accuracy or functionality of the analyte detecting members on substrate 322. As a nonlimiting example, the entire cartridge 300 may then be placed in a further sealed container such as a pouch, bag, plastic molded container, etc...to facilitate contact, improve ruggedness, and/or allow for easier handling.

In some embodiments, more than one seal layer 320 may be used to seal the cavities 306. As examples of some embodiments, multiple layers may be placed over each cavity 306, half or some selected portion of the cavities may be sealed with one layer with the other half or selected portion of the cavities sealed with another sheet or layer, different shaped cavities may use different seal layer, or the like. The seal layer 320 may have different physical properties, such as those covering the penetrating members 302 near the end of the cartridge may have a different color such as red to indicate to the user (if visually inspectable) that the user is down to say 10, 5, or other number of penetrating members before the cartridge should be changed out.

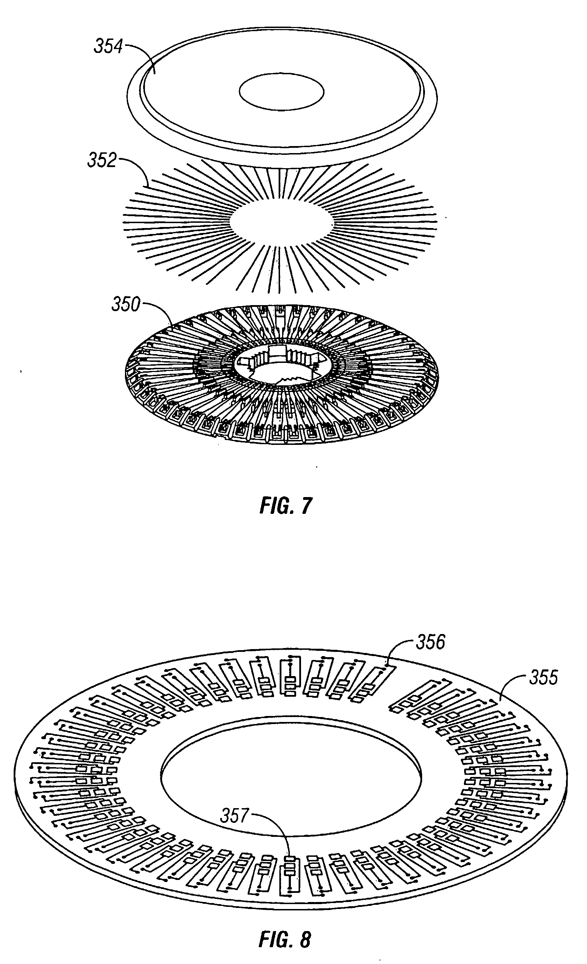

Referring now to Figure 7, an exploded view is shown of one embodiment of a cartridge 350 holding a plurality of penetrating members 352 with a heat sealed foil 354. The diameter and thickness for this one particular embodiment is shown in the figure. Of course, other embodiments may use other methods to adhere to foil 354 to the cartridge.

Referring now to Figure 8, one embodiment of layer 355 containing a plurality of analyte detecting members 356 is shown. Such a layer 355 may be adapted for use with a cartridge 350. The diameter and thickness for this one particular embodiment of the layer is shown in the figure. The analyte detecting members 356 may be coupled to contact pads 357 and electrically conductive leads. Some embodiments may use three contact pads per chamber.

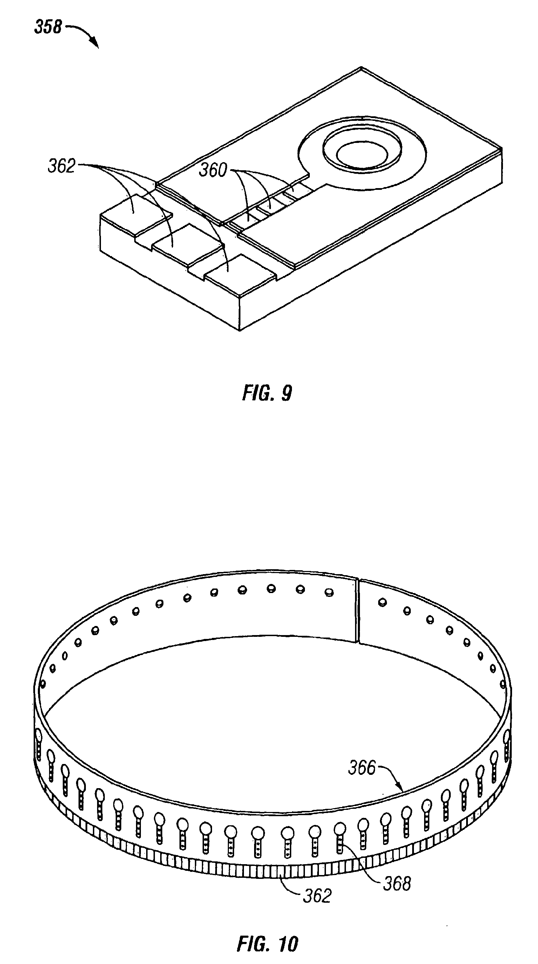

Referring now to Figure 9, one embodiment of an individual testing member 358 is shown. The member 358 includes a plurality of electrodes 360 and contact pads 362 electrically coupled to each other. The dimension for this one particular embodiment of the member is shown in the figure.

Referring now to Figure 10, one embodiment of a ribbon 364 containing a plurality of analyte detecting members 366 on the ribbon 364 is shown. In this embodiment, 50 analyte detecting members 366 may be included on the ribbon 364. It should be understood that different numbers of members such as but not limited to 10, 20, 25, 30, 40, 50, 60, 70, 75, 80, 90, 100, or more may be on a ribbon 364 and positioned on or around a cartridge containing a plurality of penetrating members. The ribbon may have electrical contact pads 362 that are on an outward surface of the ribbon 364, on an inner facing surface of the ribbon, or some contact pads may face the outer circumference while other face the inner circumference. The dimension for this one particular embodiment of the ribbon 364 is shown in the figure. The ribbon 364 may also include a vent 368 with each analyte detecting member to facilitate fluid flow.

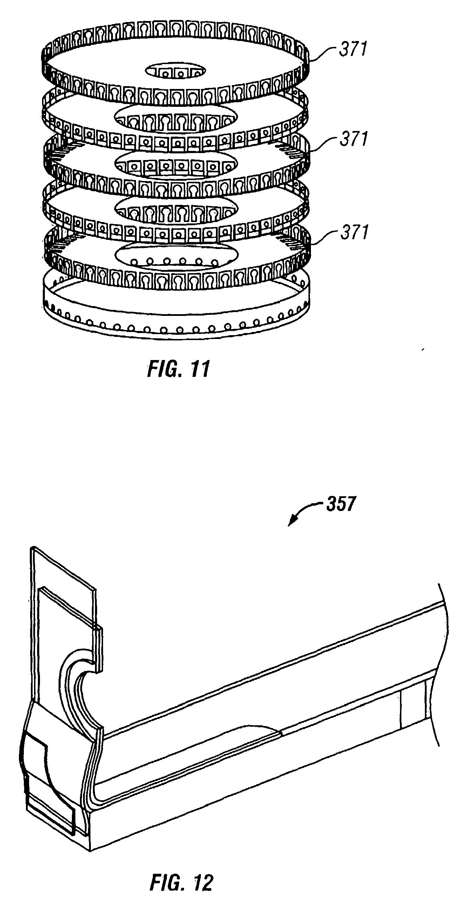

Referring now to Figure 11, one embodiment of a disc 370 having a plurality of analyte detecting members formed from a plurality of layers of materials. As seen in Figure 11, the various layers may be held together by layers 371 of PSA or other adhesives. In this embodiment, the PSA layers 371 are used to join layers of hydrophilic file, hydrophilic mesh, and hydrophilic firm.

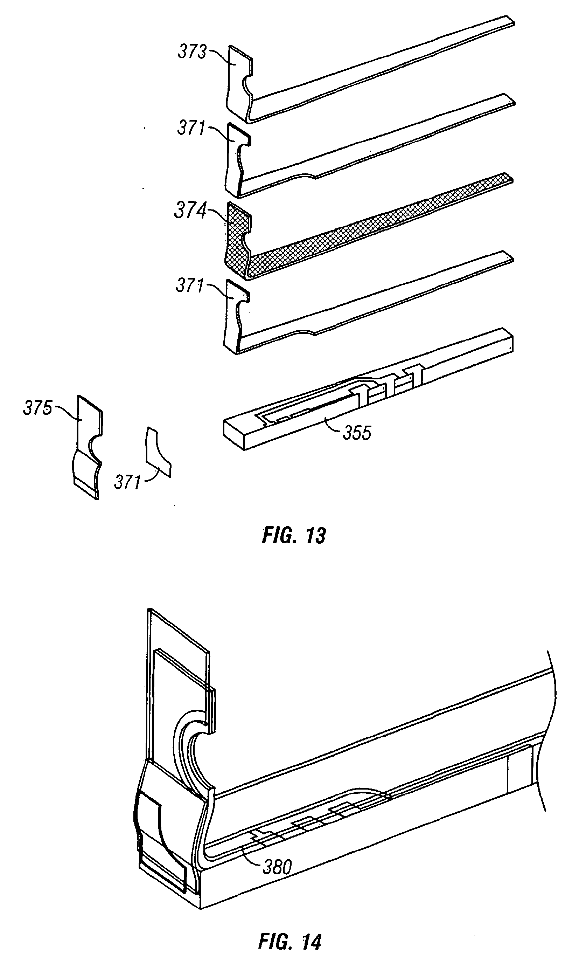

Referring now to Figure 12, one portion of the disc 370 is shown. It should be understood that there are a variety of ways of putting the capture structure together and the disc 370 shows only one embodiment for doing so. The various layers are more clearly shown in Figure 13. Again, the dimension for this one particular embodiment of the ribbon 364 is shown in the figure.

Referring now to Figure 13 shows an exploded view of the embodiment of figure 12. This embodiment includes a full mesh layer 374. The full mesh layer capture blood quickly blood from wound easily. Mesh transports blood to the analyte detecting members quickly. Layers of PSA 371 are used to couple the layers together. Hydrophilic films 373 and 375 are coupled together and then to the substrate 355. The layer 375 may be curved or shaped as desired to optimize flow.

Referring now to Figure 14, another embodiment of a sample capture structure is shown. This device may use a full capillary 380 to draw sample to the electrodes. The capillary 380 may be defined by the various layers of material 371, 373, 375, etc....

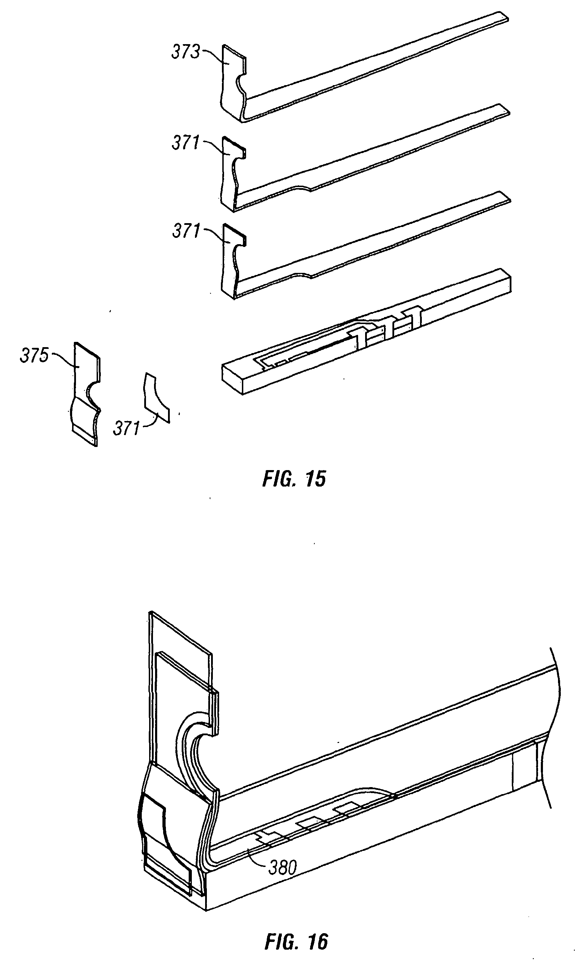

Referring now to Figure 15, an exploded view of the embodiment of Figure 14 is shown. In some embodiments, a single thick PSA layer may be used to adhere layers but also to create sufficient space to form the capillary. As seen, the PSA layers 371 include cutouts to define the capillary area 380.

Referring now to Figure 16, a still further embodiment of a sample capture structure is shown with a hybrid approach using both a mesh and a capillary to guide fluid to the desired location. Capillary area 380 remains. A mesh is used to bring fluid to this capillary area.

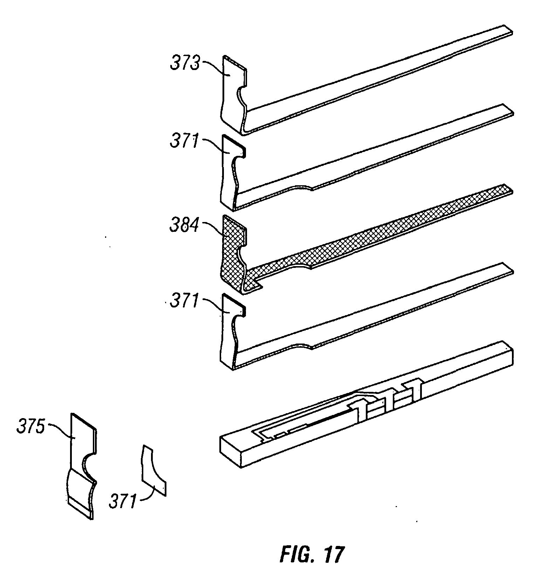

Referring now to Figure 17, an exploded view of the embodiment of Figure 16 is shown. The mesh layer 384 may be used to engage the blood or body fluid initially and then a capillary defined by area 386 may be used to draw fluid over the electrodes. Again PSA or other adhesive layers 371 of material may be used to join the various layers hydrophilic films or mesh.

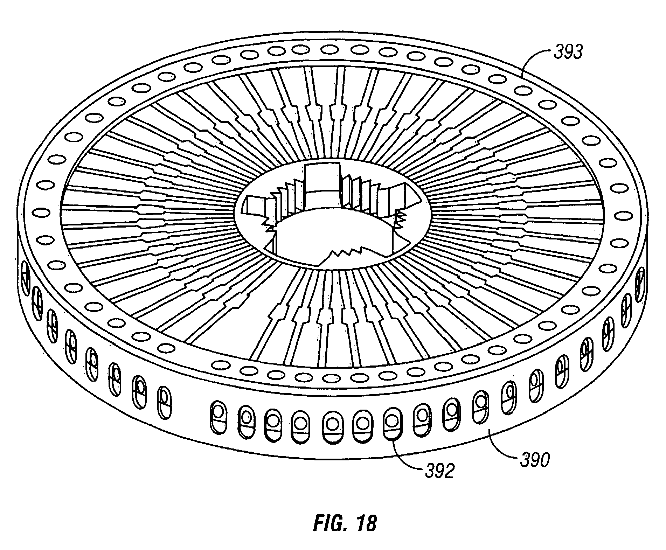

Referring now to Figure 18, a perspective view is shown of a cartridge for use with a sample capture device having a plurality of analyte detecting members. As seen in Figure 18, the outer ring 390 includes a plurality of openings 392. The openings 392 may be sized to function as reservoirs to hold excess blood or to control blood from spreading from one area to another on the ring. The openings 392 may be sized greater than the opening of the penetrating member exit from the cartridge.

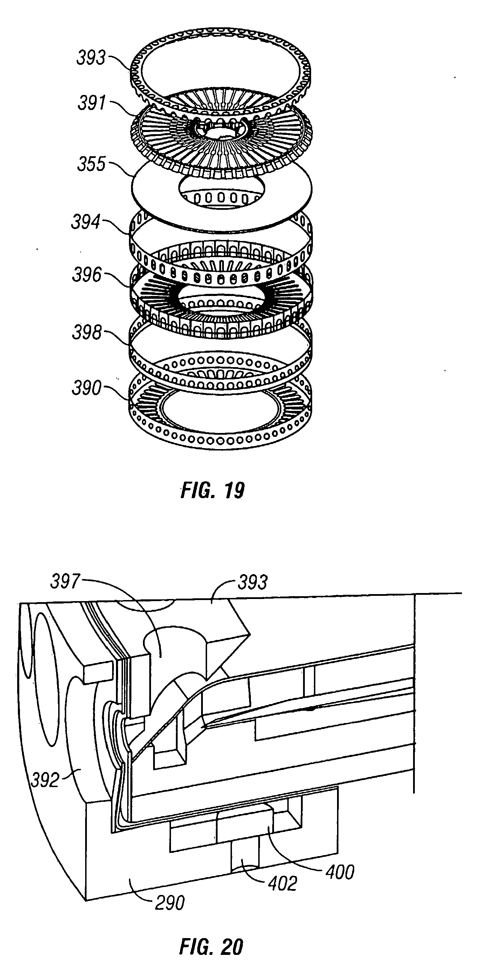

Referring now to Figure 19, an exploded view of various layers of the sample capture and analyte detecting member disc shown in Figure 18. This embodiment includes a inner ribbon 394 with a hydrophilic surface facing outward. The capture structures 396 may include PSA to attach to the ribbon or to the analyte detecting member disc. The capture structure on the outside may have a HY9 film that had a hydrophilic surface facing the channel and poly ethylene surface facing outward. An outer ribbon 398 may be hydrophibic and may be based on a thick piece of PSA. The ribbon 398 may have a silicone liner that provides the hydrophobic surface on the outside. Thus, a structure that is hydrophilic on the inside but hydrophobic on the outside is formed. This embodiment also includes an upside down substrate 355, a penetrating member cartridge 391, an inner ring 393, and outer ring 390 similar to that shown in Figure 18. The ring 393 may include an opening 397 to allow for a punch move substantially vertically to enter to pierce a seal on the penetrating member disc.

Referring now to Figure 20, a cross-section along one area of the embodiment of Figure 18 is shown. A desiccant 400 may be included. A vent 402 may also be included. The outer ring may provide sufficient standoff for the user's finger so that there is space for a blood droplet to form between the finger surface and the surface of the sample capture structure. The opening 392 may also act as a reservoir to trap excess blood. In this embodiment, electrical connections and vents can be made without any through connections in the analyte detecting member disc. Blood flow can be visualized. The outer ring 390 has many advantages including but not limited to finger positioning (controls distance, centers pooching) and excess blood containment, as mentioned.

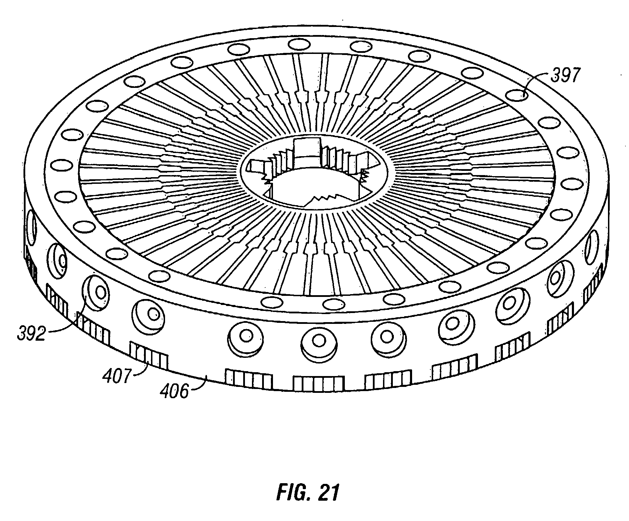

Referring now to Figure 21, another embodiment of a penetrating member cartridge for use with a sample capture/analyte detecting member devices is shown. Again, the dimension for this one particular embodiment of the invention is shown in the figure. The outer ring 406 is shown with cutout 407 to allow for the contact pads to be exposed on an outward radial surface.

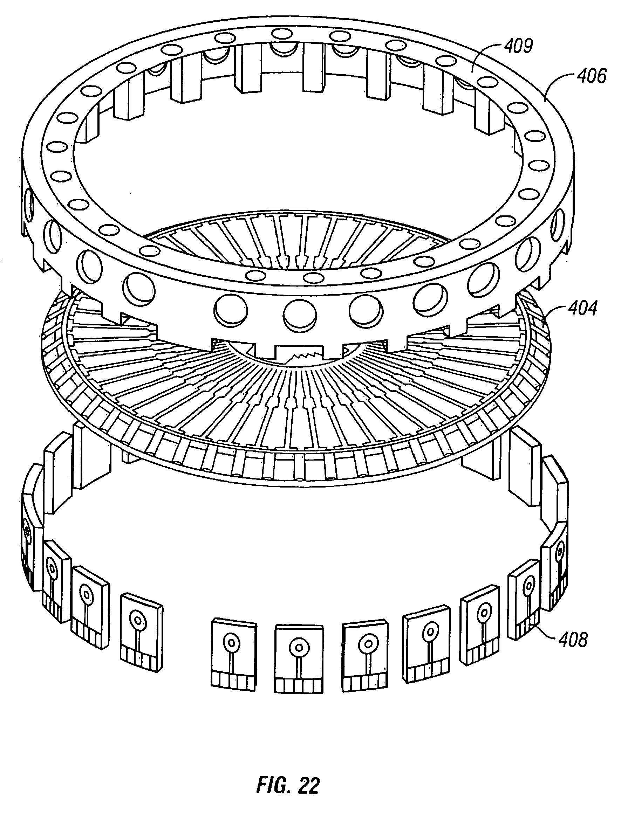

Referring now to Figure 22, an explode view of the embodiment of Figure 21 is shown. An cartridge 404 having a plurality of penetrating member is coupled to an holding ring 406 which holds a plurality of strips 408 that have sample capture and analyte detecting capability. In the embodiment for this figure, there are about 25 strips 408 for use with the cartridge. It should be understood that different numbers of strips 408 such as but not limited to 10, 20, 25, 30, 35, 40, 50, 60, 70, 75, 80, 90, 100, or more may be on positioned on or around a cartridge containing a plurality of penetrating members. Capture structure fabrication is simple. Electrical connections can be made without any through connections in the strip 408. Sealing and sterility may also be simplified since there is, in one embodiment, a plastic barrier in the outer ring between each strip 408. Inner ring 409 is used to accommodate the angled lip of penetrating member cartridge 404 and to provide an opening to allow for a punch to break a seal on the penetrating member cartridge.

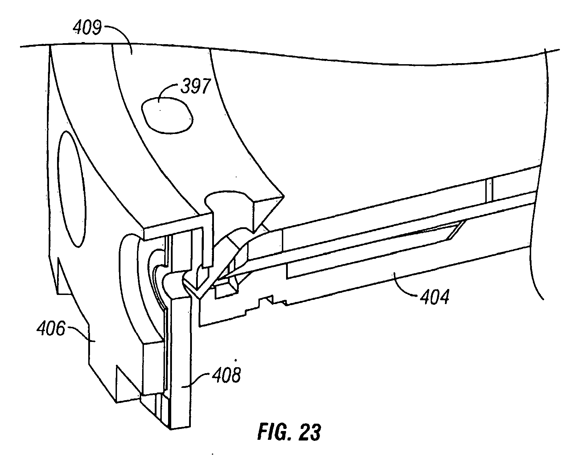

Referring now to Figure 23, a cross-section across one portion of the embodiment of Figure 21 is shown. It should be understood that in some embodiments the strips 408 may be coupled together by tape or film or other connector so that instead of being individual device which are placed one by one into position, these devices maybe placed all at once into position around the cartridge 404.

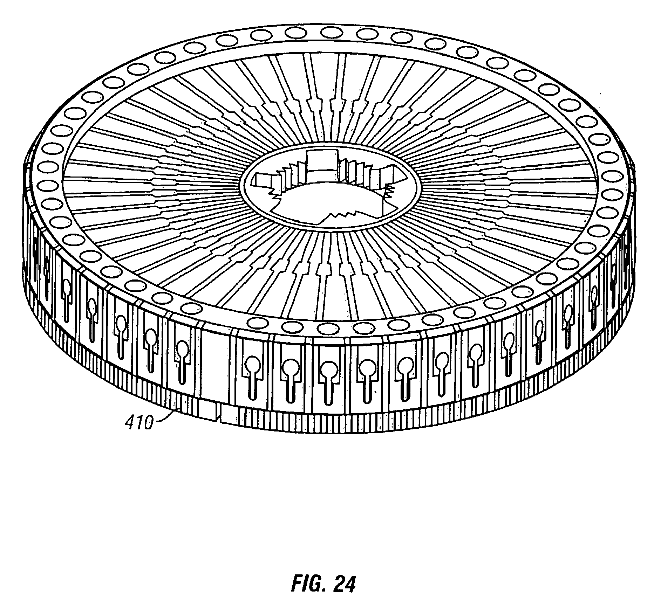

Referring now to Figure 24, another embodiment is shown similar to that of Figure 21. In this embodiment, the analyte detecting members are all mounted on a ribbon 410. Again, the dimension for this one particular embodiment of the invention is shown in the figure.

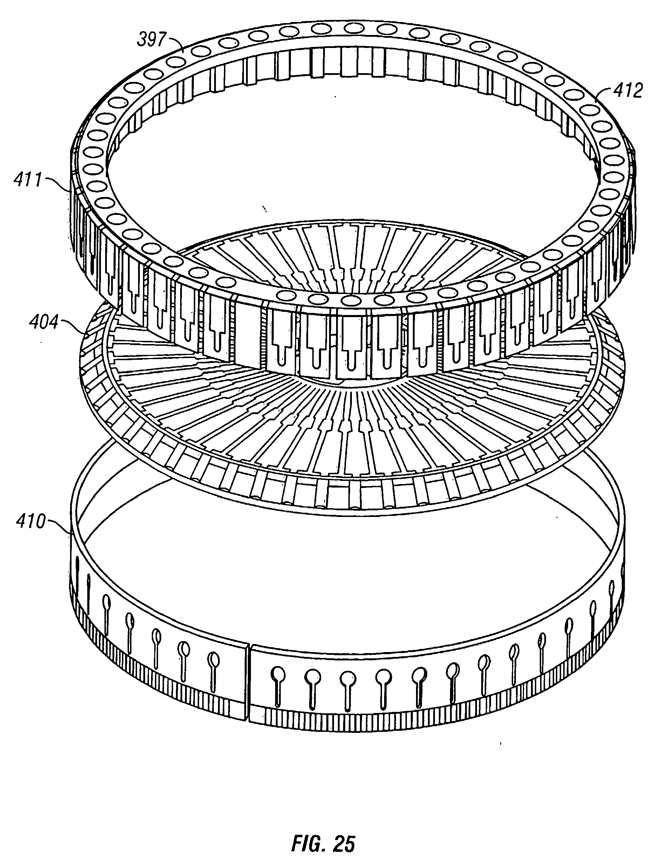

Referring now to Figure 25, this exploded view more clearly shows the individual components of the embodiment of Figure 24. A plastic ring 412 is shown that holds the ribbon 410 around the cartridge 404. The ring 412 may have openings 397 and openings 411 on a radial surface. The ribbon 410 includes a plurality of electrodes and contact pads. There are a sufficient number of electrodes to perform a plurality of tests. There i

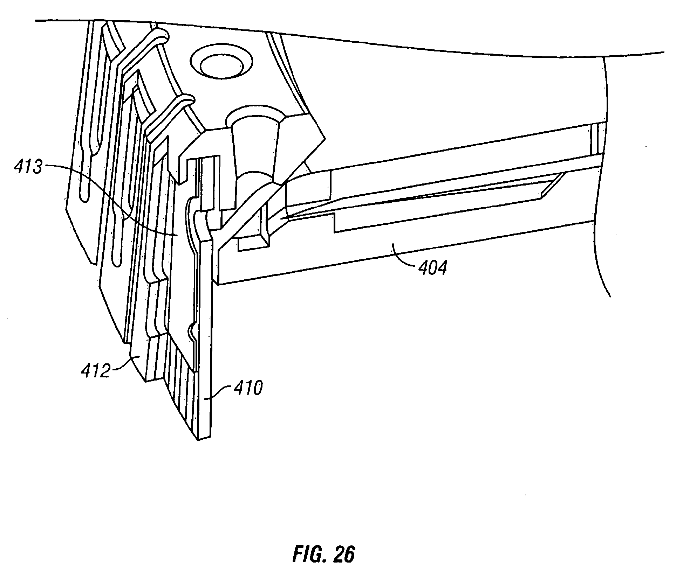

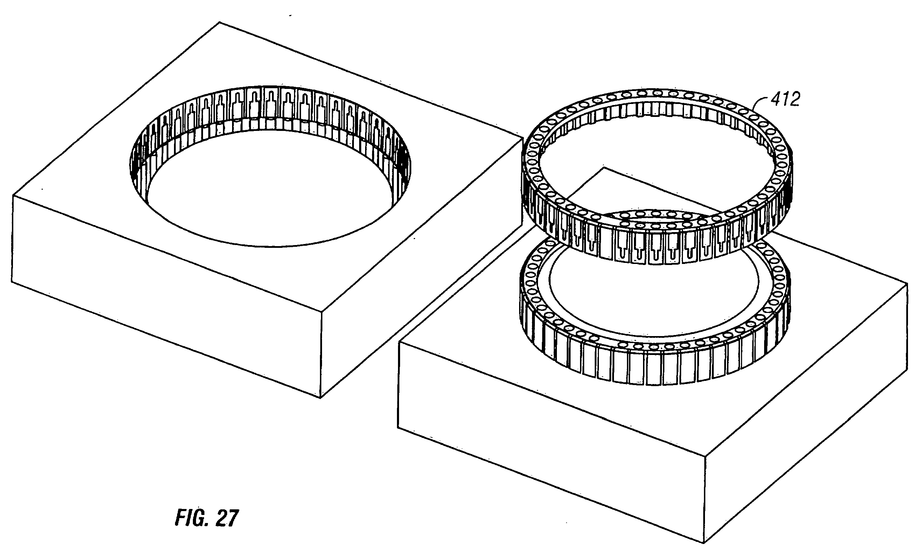

Referring now to Figure 26, a cross-section of one area of the device is shown. Electrical connections can be made without any through connections in the ribbon. Assembly is probably easier than for the mini-strips. Plastic slotted ring 412 can be easily molded as shown in Figure 27. The ring 412 may include a slot 413 for receiving the ribbon 410. The slot 413 may extend continuously around the ring 412. Some embodiments may have the slot extend around portions of the ring 412.

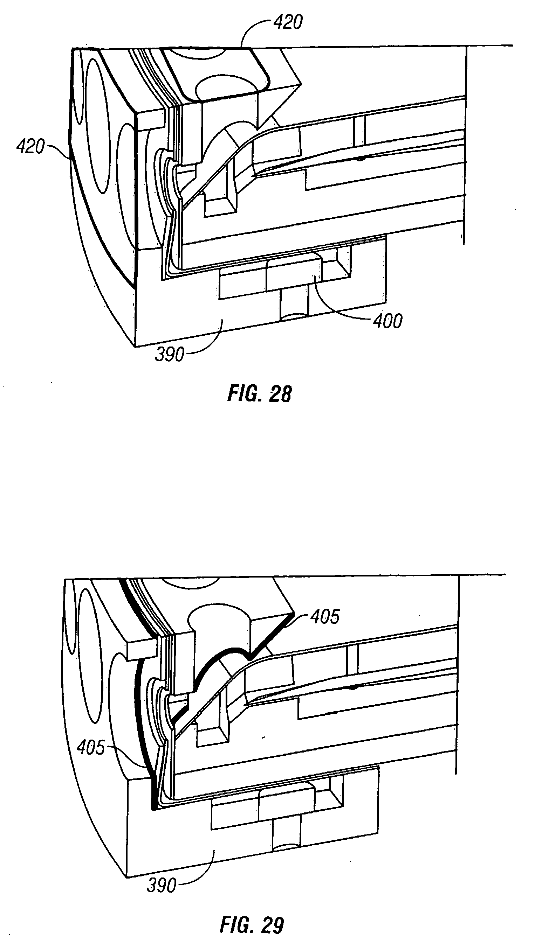

Referring now to Figure 28, sealing and sterility issues will now be discussed. Figure 28 shows seals attached to the device shown in Figure 20. The seals 420 may be two separate pieces that may be heat sealed (in one embodiment) to the ring 390. A standoff from the capture surface may be used in some embodiments so that the foil can be removed without damaging the capture structures. In one embodiment, this standoff can be created by using plastic "ribs". The space between these "ribs" and the capture structures will then also need to be sealed.

Referring now to Figure 29, sealing of the backside of the ring 390 to the cartridge 404 is shown. Adhesive 405 can fill gaps (either by flowing or by being squishy), thus taking care of tolerance issues.

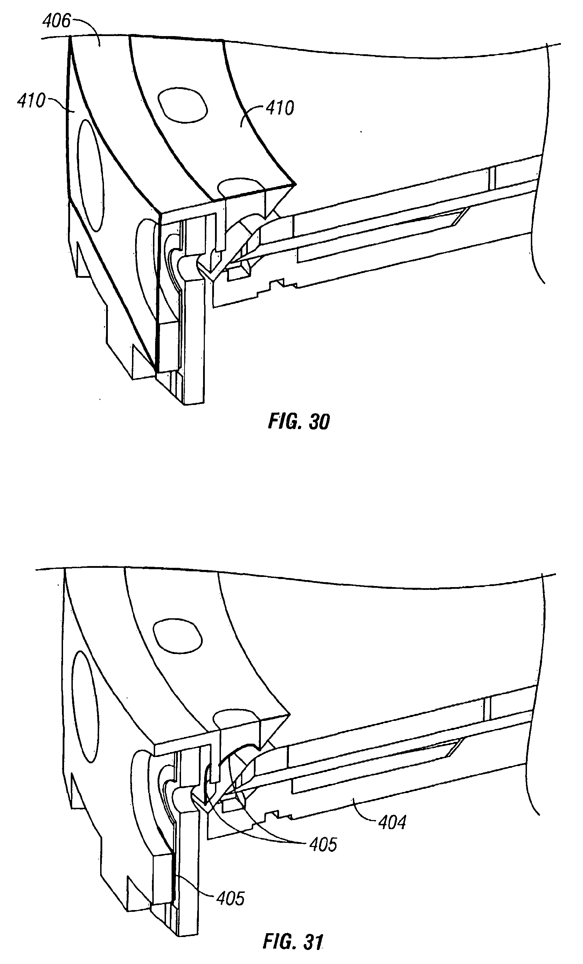

Referring now to Figure 30, foil 410 is shown on an embodiment as shown in Figure 22.

Referring now to Figure 31, the adhesive 405 may also be used on the back side to couple to a cartridge 404.

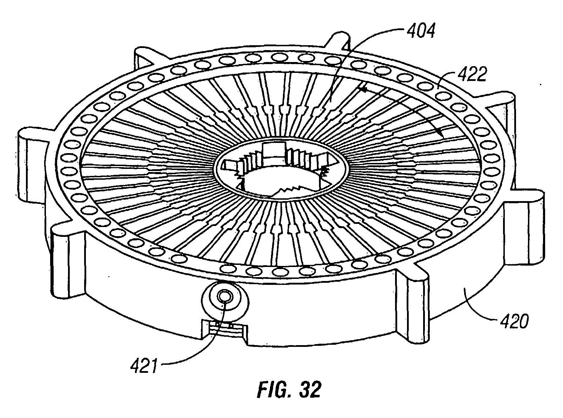

Referring now to Figure 32, a still further embodiment is shown for sealing the analyte detecting members. In this embodiment, there is a tight fit between an outer ring 420 and an inner ring 422. The tight fit may be sufficient to create an air-tight seal over those devices that are not under the opening 421. As indicated by arrow 424, the inner ring 422 an rotate while the outer ring 420 stays stationary. In some embodiments, the ring 420 rotates while the inner ring 422 and cartridge 404 stays stationary. Again, the dimension for this one particular embodiment of the invention is shown in the figure.

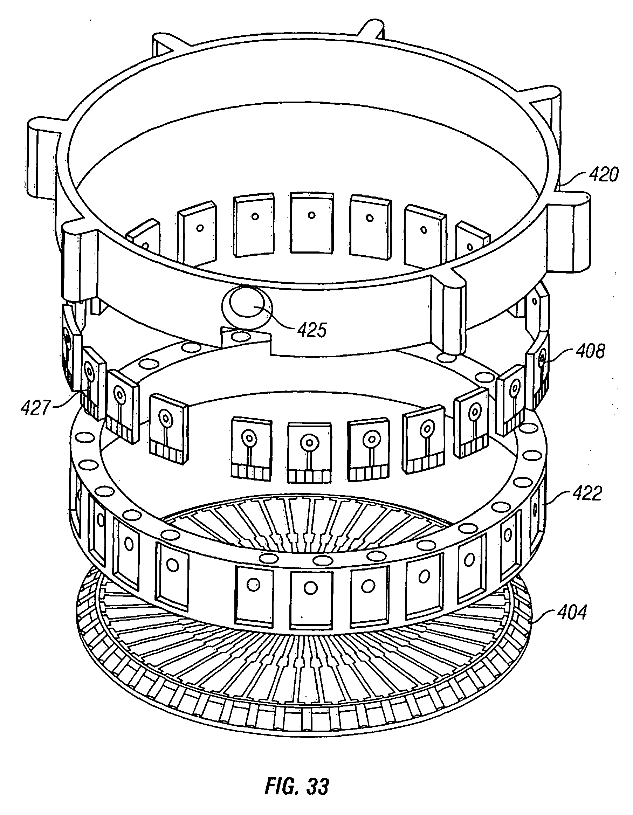

Referring now to Figure 33 shows an exploded view of the various elements of the embodiment of Figure 32. The outer ring 420 is of sufficient tolerance to provide a sterility seal to protect strips 408 when those strips 408 are not in an active position. As seen in Figure 33, there may be a blank area where no strip 408 is located so that all strips can be sealed when the device is initially used. When the strip 408 is rotated to align with hole 425, then the strip is unsealed and in an active position. It should be understood that this ring may be adapted for use with any of the embodiments described herein. In one embodiment, foil is not used when the device uses the ring shown in this figure. The inner ring 422 is shown with recesses 423 to hold the test elements 427.

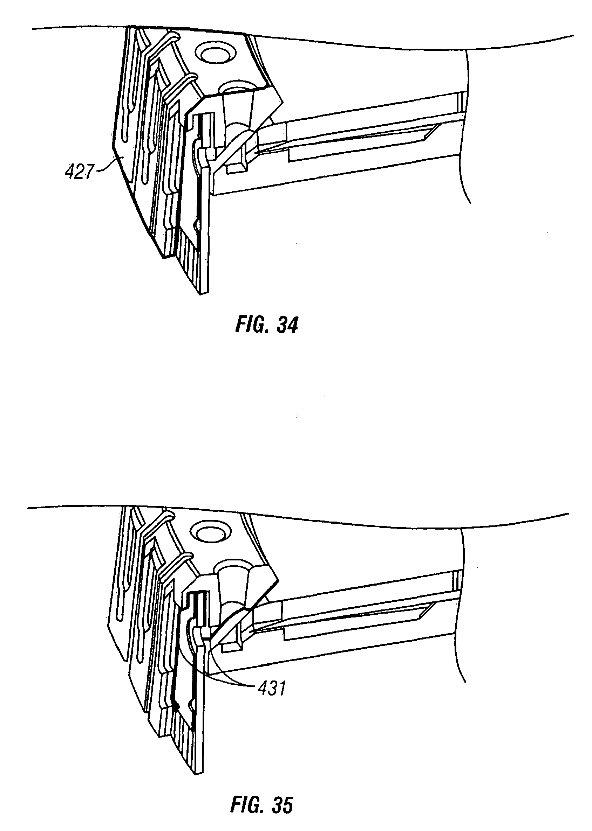

Referring now to Figure 34, the embodiment of Figure 24 is shown with a single foil layer427 that covers both lateral and top openings on the outer ring.

Referring now to Figure 35, the gap-filling adhesive 431 is shown to seal pathways near the backsides of the ribbon.

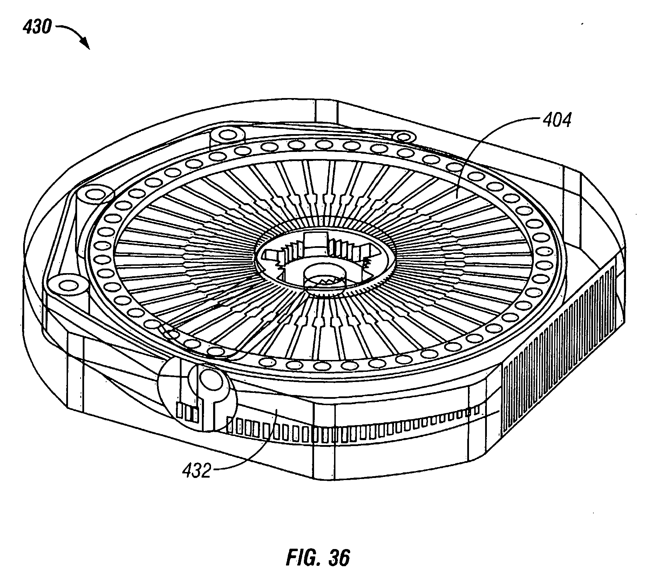

Referring now to Figure 36, a still further embodiment is shown. A housing 430 is shown with a cartridge 404 with a tape layer that covers the outer circumferential surface of a ribbon 432 with a plurality of analyte detecting members. Again, the dimension for this one particular embodiment of the invention is shown in the figure. The tape may be a moisture and air impermeable film. In one embodiment, the spool may have a diameter of 20.0 mm and wind up 3.4 mm of film exposing one and only one sensor.

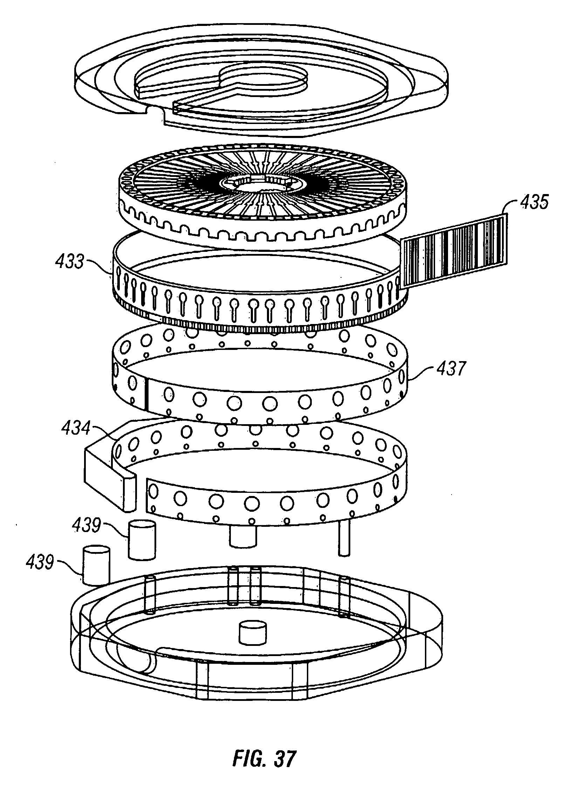

Referring now to Figure 37, an exploded view of the embodiment of Figure 36 is shown. A film 434 is around the circumference, covering the ribbon 433 and providing sterility. The film 434 is peeled back to uncover one or more analyte detecting member on the ribbon. Most embodiments uncover only one. A bar code 435 may be provided on the housing to provide information to the device. The cover film 434 may be an outer layer. Another embodiment may include an inner layer 437. Pulleys 439 are arranged to allow the outer film 434 to be pulled back.

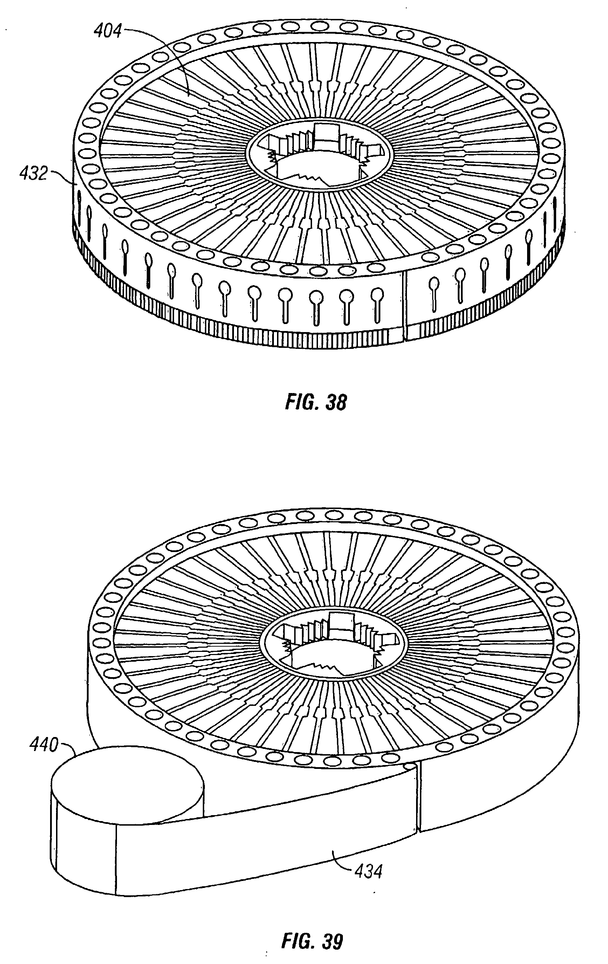

Referring now to Figure 38, the concept of the peeling of the film is shown. The ribbon 432 is positioned about a penetrating member cartridge 404.

Referring now to Figure 39, a sterility film 434 is provided to release each analyte detecting member to bring it to an active position. A spool 440 may be used to store cover film 434 as it is detached from the ribbon.

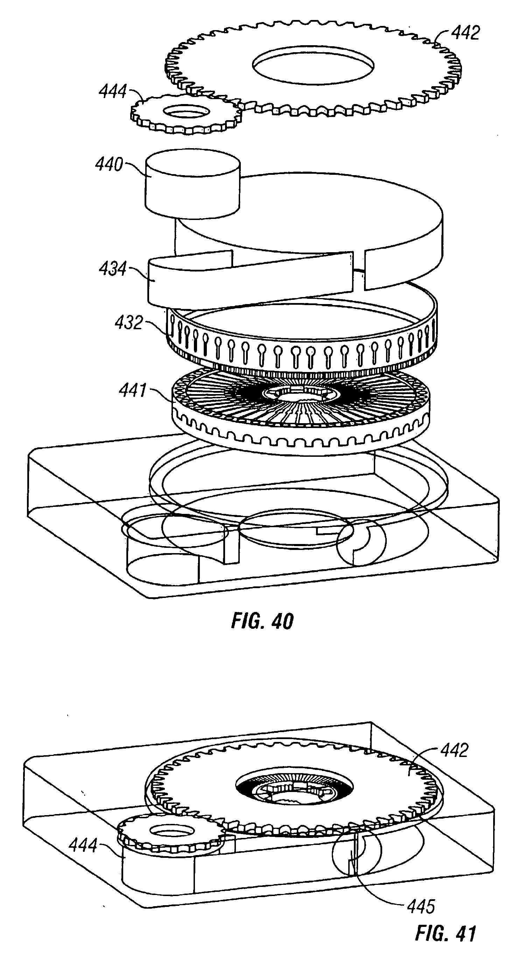

Referring now to Figure 40, an exploded view of the various components in a housing. The spool 440 and the cartridge may have gears 442 and 444 to coordinate the motion of the penetrating member cartridge 441 and the spool 440.

Referring now to Figure 41, an assembled device of Figure 40 is shown. There is an opening 441 to allow the penetrating member to exit.

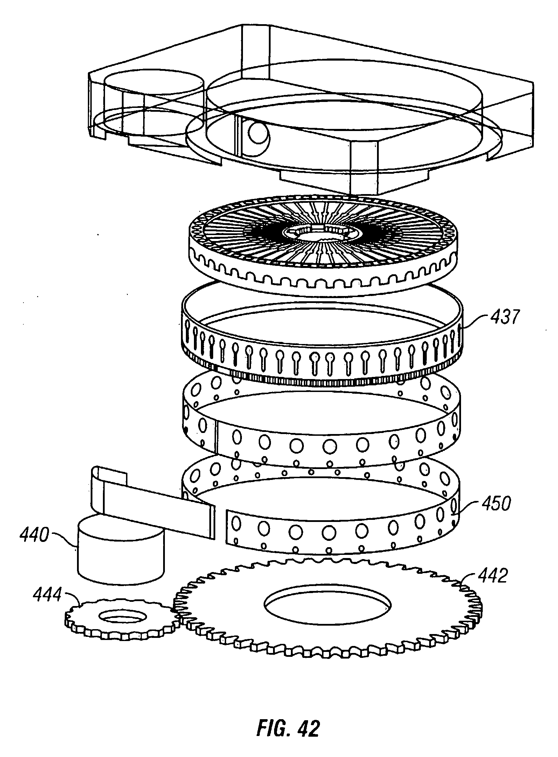

Referring now to Figure 42, a further embodiment may have the film 450 wrapped twice around the ribbon. This embodiment has a film 450 with holes so that only every other analyte detecting member is uncovered when the film is peeled. Thus, the openings on the film are spaced apart to be offset by one. This allows for more opening or space between where the pulley is located and the area where uncovered analyte detecting member is located. As the second layer is removed, the other set (odd or even) of analyte detecting members are uncovered. The device may rotate two "spots" but only one new analyte detecting member is uncovered.

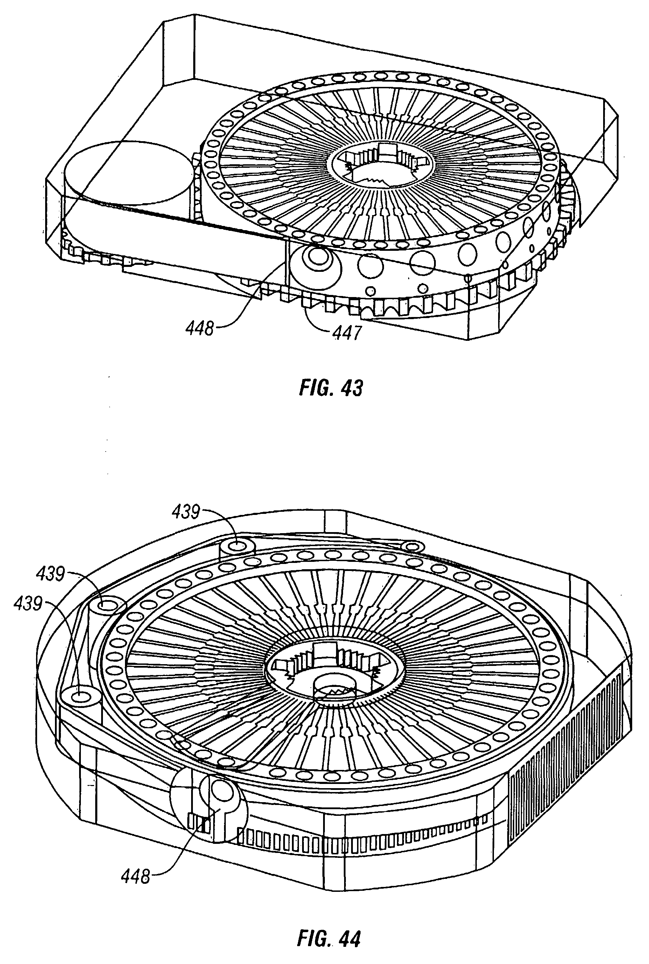

Referring now to Figure 43, one embodiment of the assembled version of device of Figure 42 is shown. Again, the dimension for this one particular embodiment of the invention is shown in the figure. As seen, the gears 442 and 444 are located on an underside of the device. There is also an opening 447 that allows the gears to be accessed. An opening 448 is provided for penetrating member exit from the housing.

Referring now to Figure 44, another embodiment of the present invention is shown. In this embodiment, the housing is more compact as there is no spool. Here the film that is removed is then reattached to a back side of the cartridge. Again, the dimension for this one particular embodiment of the invention is shown in the figure. This embodiment is similar to that of Figure 37. Pulleys 439 are used.

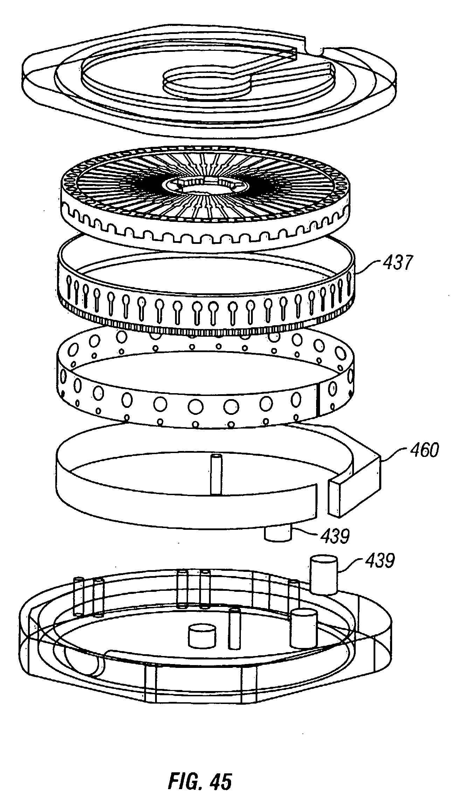

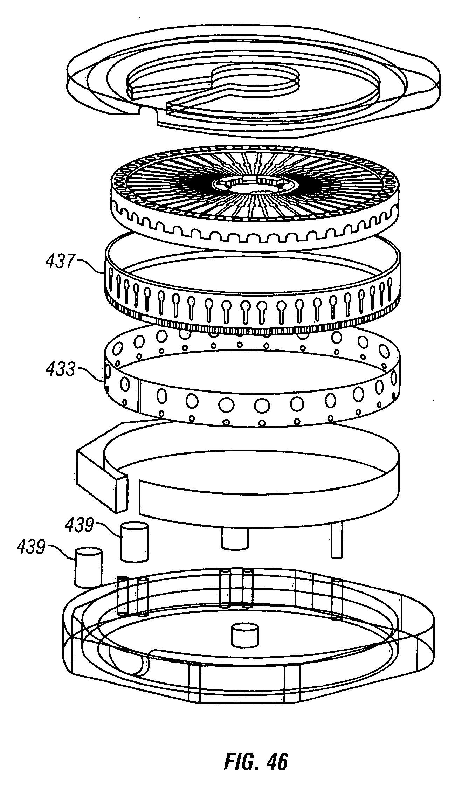

Referring now to Figure 45, an exploded view of the embodiment of Figure 44 is shown. The rollers or pulleys (Figure 46) are used to route our portion 460 the detached film to reattach the film to the backside.

Referring now to Figure 46, another exploded view of the embodiment of Figure 44 is shown. There are no gears. In one embodiment, the rollers are not attached or driven. The tension of film should naturally turn with the motion of the film. The peel film embodiment does not need to seal/punch an external foil. This embodiment does not need a gap-filling adhesive. Sealing can be done with both the Ribbon and the Strip. Assembly of wound ribbons in a cartridge is widespread.

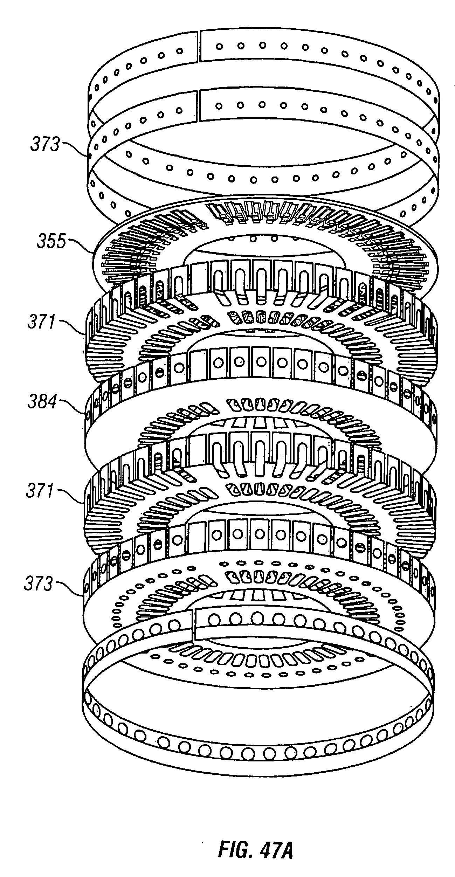

Referring now to Figure 47A to 47C, various embodiments of the present invention are shown.

Figure 47A shows a disc based device. The disc is a substrate 355 with a plurality of analyte detecting members. There are a plurality of PSA layers 371, hydrophilic mesh 384, and hydrophilic films 373 (such as but not limited to HY-9).

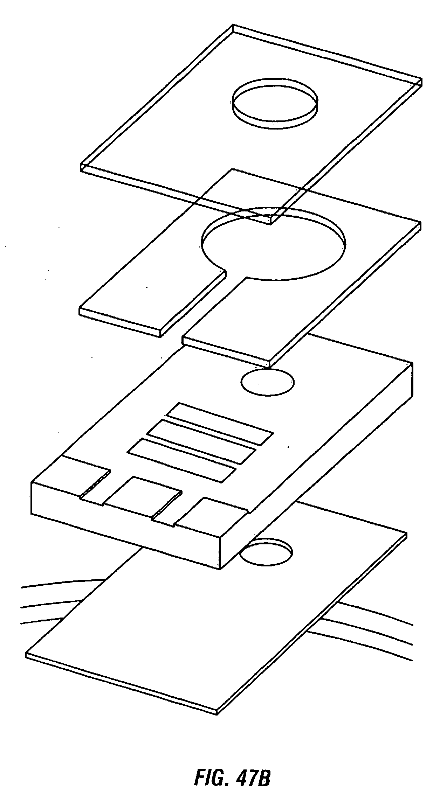

Figure 47B shows an embodiment built on individual strips 408 as seen in Figure 22.

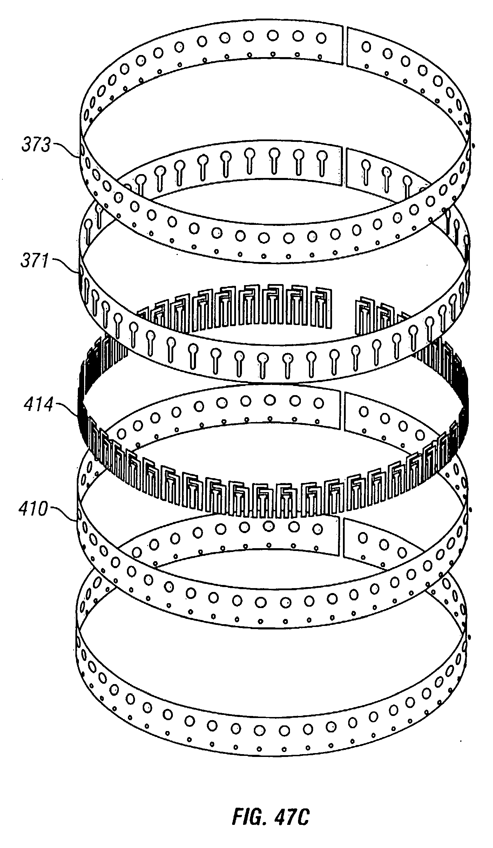

Figure 47C shows a configuration based on ribbon 410 with electrical contact pads and electrodes 414.

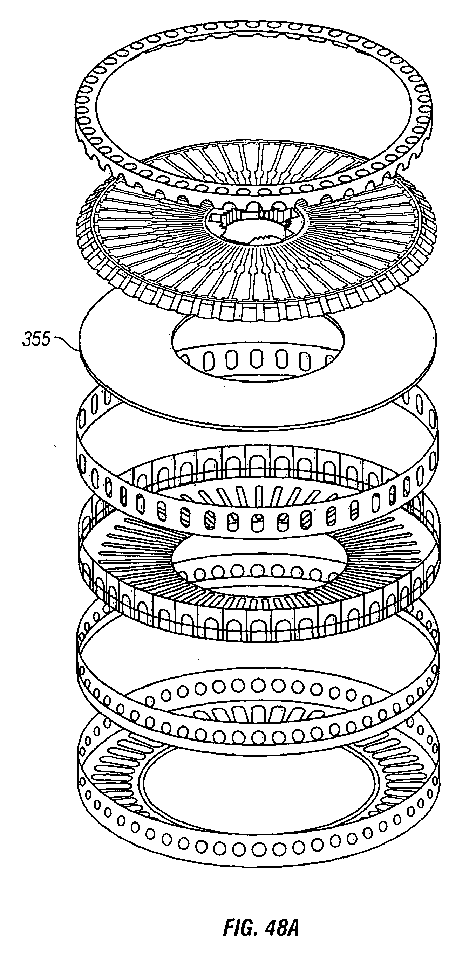

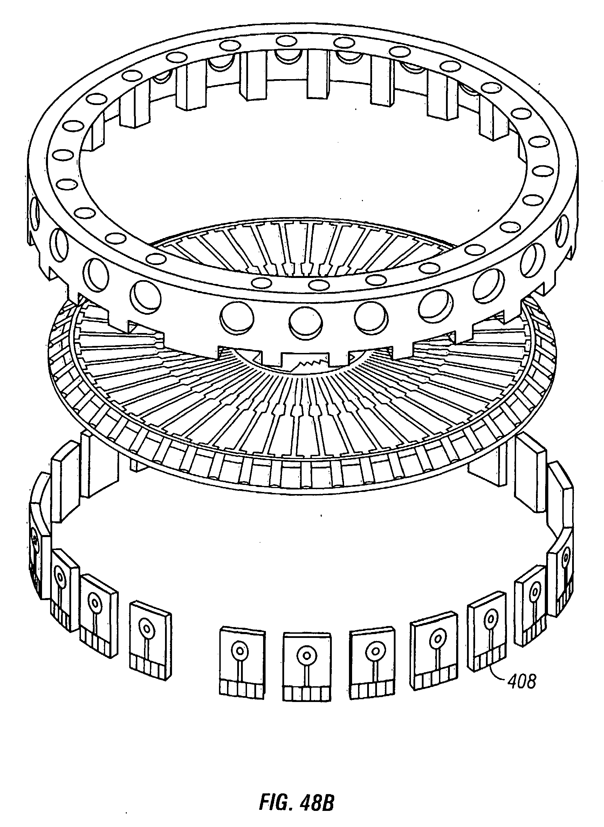

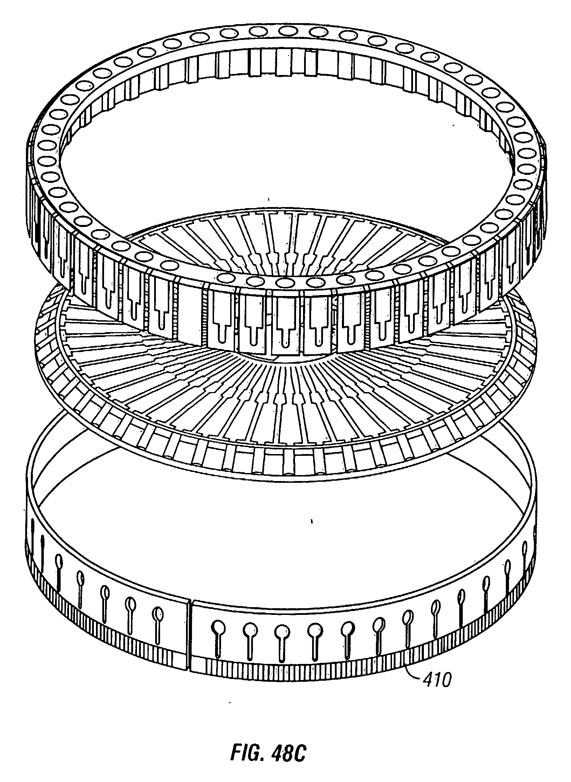

Referring now to Figure 48A to 48C, various methods of placing analyte detecting members around a cartridge 404 is shown. Figure 48A shows the disc 355 with analyte detecting members. Figure 48B shows the use of the strips 408 with the present invention. Figure 48C shows the use of a ribbon 410 with a disposable.

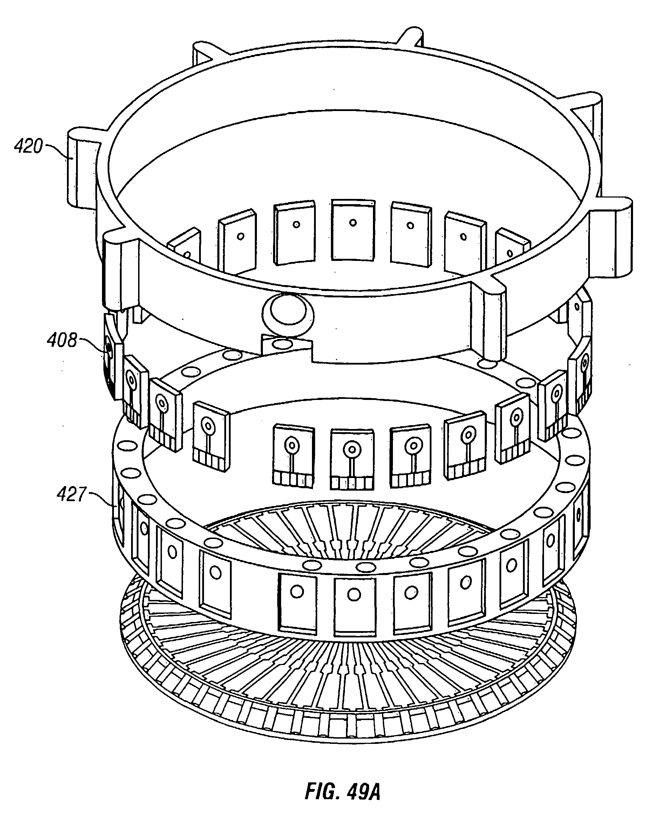

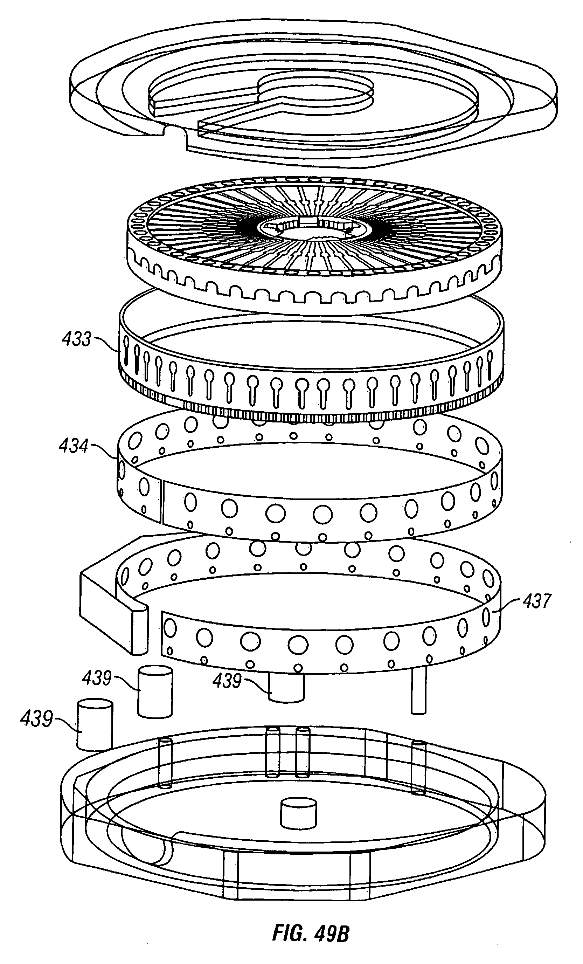

Referring now to Figure 49A and 49B, other methods of sealing using the rotating, sealing ring and a peeling film are shown respectively.

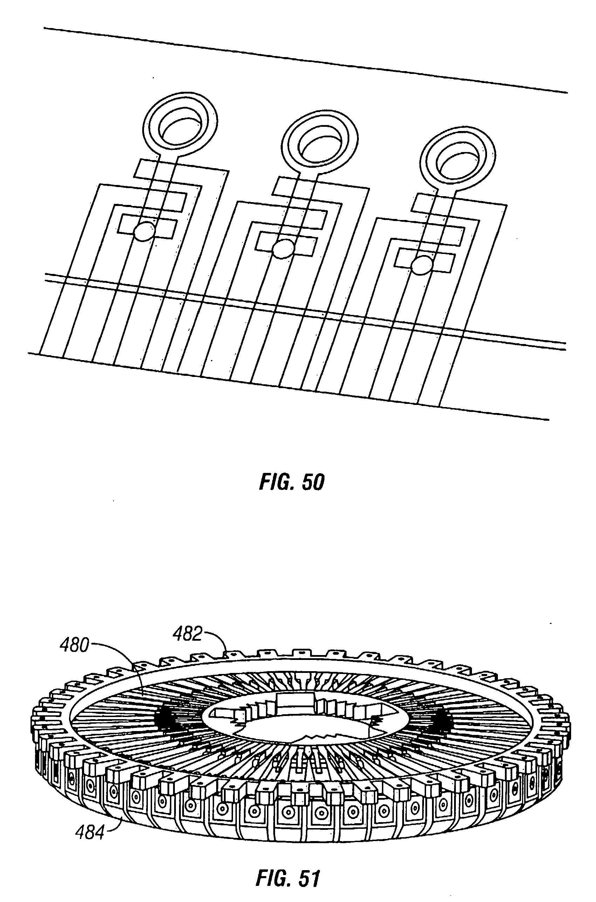

Referring now to Figure 50, one embodiment of a ribbon having a plurality of analyte detecting members is shown. This embodiment is purely exemplary and is nonlimiting.

Referring now to Figure 51, yet another embodiment of the present invention will now be described. This embodiment of the invention includes cartridge 480 that holds a plurality of penetrating members. A support ring 482 is coupled to the disc shaped cartridge 480 to hold a plurality of analyte detecting members 484 to the cartridge 480. The members 484 may be formed on flat sheet and then have the individual "fingers" of the analyte detecting member to be cut into the flat sheet along a circumference such as but not limited to laser cutting so that the fingers may bent up as shown in Figure 51.

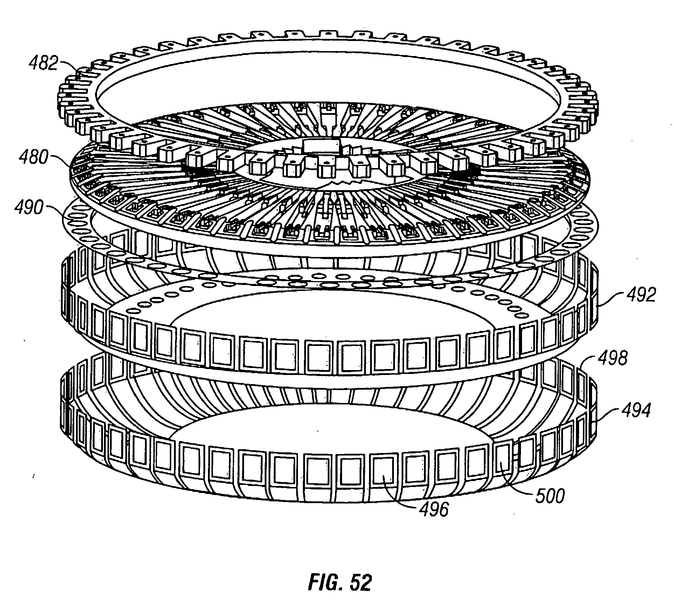

Referring now to Figure 52, an exploded view of the embodiment of Figure 51 is shown. As seen, a layer of PSA 490 maybe used to attach an inner foil layer 492 to the cartridge 480. The foil layer 492 may be heat sealed to the inner surface of the frame layer 494 having the analyte detecting members 484. Individual foils 496 may be coupled to the outer circumferential surface. As seen in Figure 52, frames or carve outs are provided on the inner surface at location 498 for example and on the outer position at location 500 to allow for sufficient set back that the foil can be puncture or pushed down by a plough or double plough device.

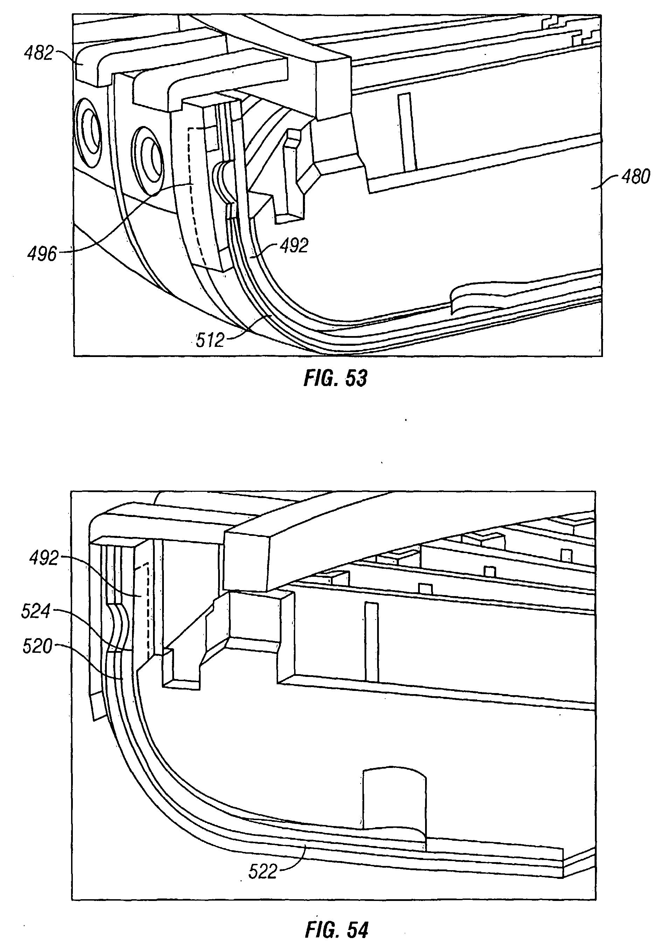

Referring now to Figure 53, a cross-section of the embodiment of Figure 51 is shown. The foil 496 and foil 492 provide a sealed environment for the analyte detecting member. Desiccant may be provided in a desiccant cavity 510. There is a blood channel 512 in the analyte detecting member.

Referring now to Figure 54, it can be seen that a vent channel 520 provides for venting from port 522 to the upper area 524 to be opened by the foil 492 is opened. This allows for the opening a vent that is not on the underside of the cartridge.

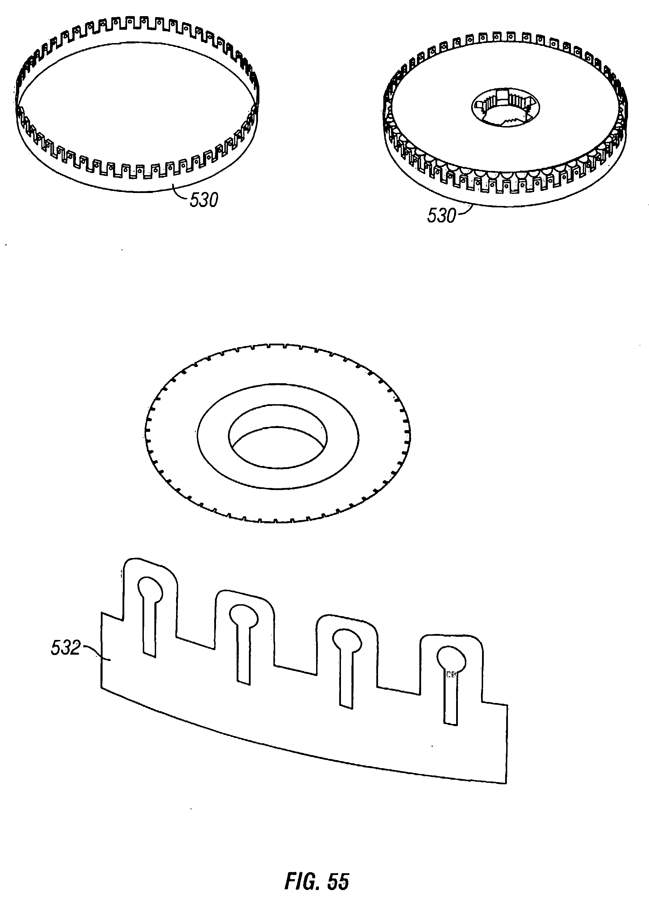

Referring now to Figure 55, yet another embodiment of the present invention will now be described. Dissolvable films have been developed for applications such as cosmetics, oral therapeutic agents, and food products. The unique properties of these materials include a finely tunable dissolution rate, and the ability to alter the surface texture. Additionally, the thickness of the film can be controlled to relatively tight tolerances. These films can function after prolonged exposure to typical environmental conditions, and can be surface treated to withstand higher levels of prolonged humidity.

The electrochemical analyte detecting member used with the present invention may be sensitive to moisture, which can inactivate the sensor. In one embodiment, a hermetic seal is not required for these sensors, only a hygroscopic seal. This makes the invention of a dissolvable seal a means to simplify the design.

In embodiment, these films are generally starch-based, and the presence of certain enzymes in the blood could break down dissolved starch into glucose. This reaction could be a significant source of signal in the electrochemical detection of glucose. This response can be corrected for, or minimized in the design of the sample capture structure to minimize this as a source of signal error.

In one embodiment, this film also offers the possibility of a top load sensor that minimizes the required volume. Rather than requiring a discrete, fully formed channel, the dissolution can be tuned so that the electrochemical reaction is triggered on the condition that electrical continuity among the sensor electrodes is detected. The trigger may be timed with the known dissolution properties and sample capture surface designed to achieve a consistent signal response. This would obviate the need for a channel vent, and reduce the sample volume requirements.

Figure 55 shows an assembly with a lancing component (silver), an outer sensor strip 530 (orange). The fluidic channel is made with a structural adhesive 532 (black) connected to the orange sensor strip 530. It is principally topped with a non-dissolvable film. Selected areas defined as the sample capture surface are topped with the dissolvable film.

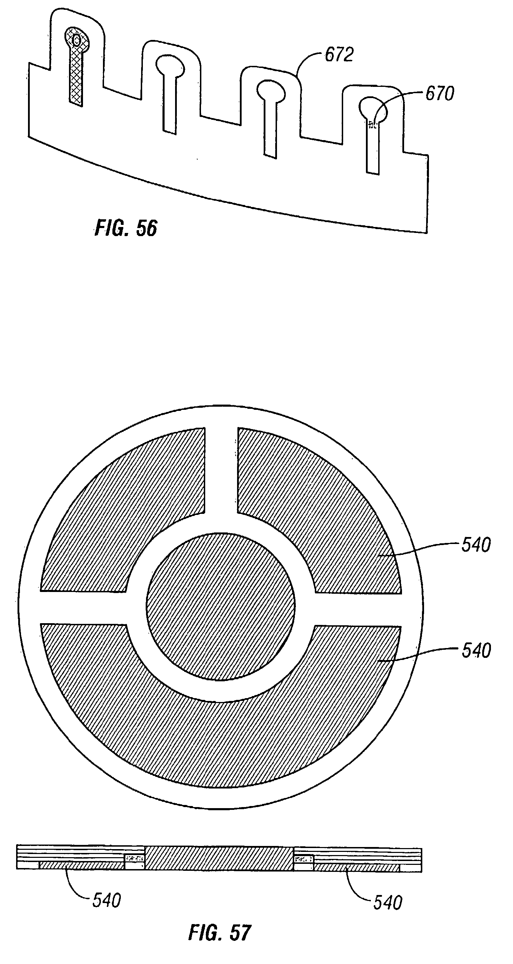

Referring now to the embodiment of Figure 56, this figure is a larger image of the structural adhesive in Figure 55. It depicts two possible locations of the dissolvable film at location 670 and 674.

Referring now to the embodiment of Figure 57, a top loading configuration with a lancet aperture that passes through the analyte detecting member. The sensor electrodes 540are in close proximity to the penetrating member aperture. They are radially arrayed in this configuration.

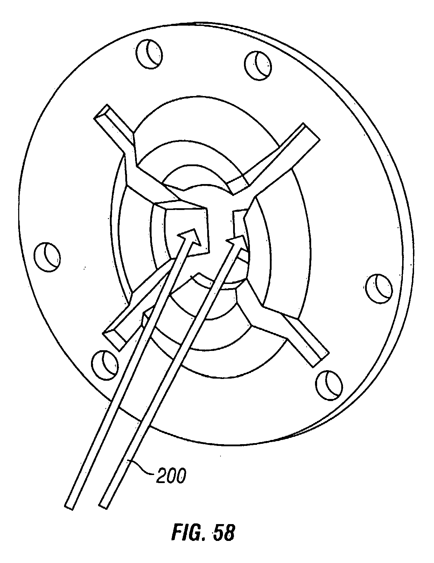

Referring now to Figure 58, yet another embodiment of the present invention will now be described. With any penetrating member actuator assembly, there is a location where the penetrating member interfaces the skin. This interface, or "front end interface" is usually a surface that is flat or contoured to accept a suitable skin lancing site, such as the finger. Within this surface is an opening of various sizes or shapes that the penetrating member passes through when piercing the skin.

To combine the lancing and sensing steps into an integrated, seamless process, a spontaneously derived blood sample on the skin surface is captured with a fluidic system collocated with the penetrating member insult. It is an advantage to have a miniaturized integrated disposable test cartridge that allows for several test units to be placed within close proximity next to each other.

The each wound bleeds differently from one lancing event to the next. The relative location and size of the blood droplet should be well suited to the sample capture surface. In the cases when the droplet is not well controlled, a secondary structure that prevents blood movement across the test cartridge onto an unused unit is desirable to eliminate the risk of cross contamination.

To solve this problem, the invention is a rubberized penetrating member/skin front end interface that forms a seal when the finger, or other lancing site, depresses the structure. Finger pressure bends the front end interface inwards and the back surface of the interface mates to the test cartridge and forms a seal. The seam of this seal forms a perimeter around the active sample capture unit.

In the event that there is too much blood, the blood is confined to the active sample capture unit. Excess blood is captured by the front end interface. The front end interface is removable and cleanable without effecting the rest of the device or the test cartridge.

In this manner the lancing site is consistently positioned relative to the penetrating member's travel while allowing the multiple unit cartridge to be freely repositioned when the user is not depressing the interface.

Figure 58 shows one embodiment of the front end. The feature that seals the active sensor from the adjacent sensor is highlighted by arrows 700.

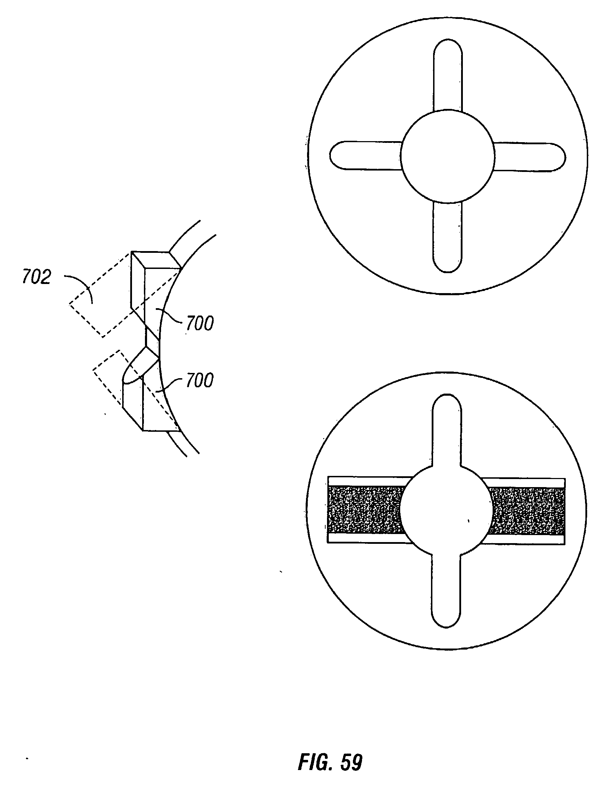

Figure 59 shows that the portions highlighted by arrows 700 may engage mating portions 702 shaped to receive the portions identified. This may be trough or valley area that will trap an excess fluid in the area and prevent them from spreading.

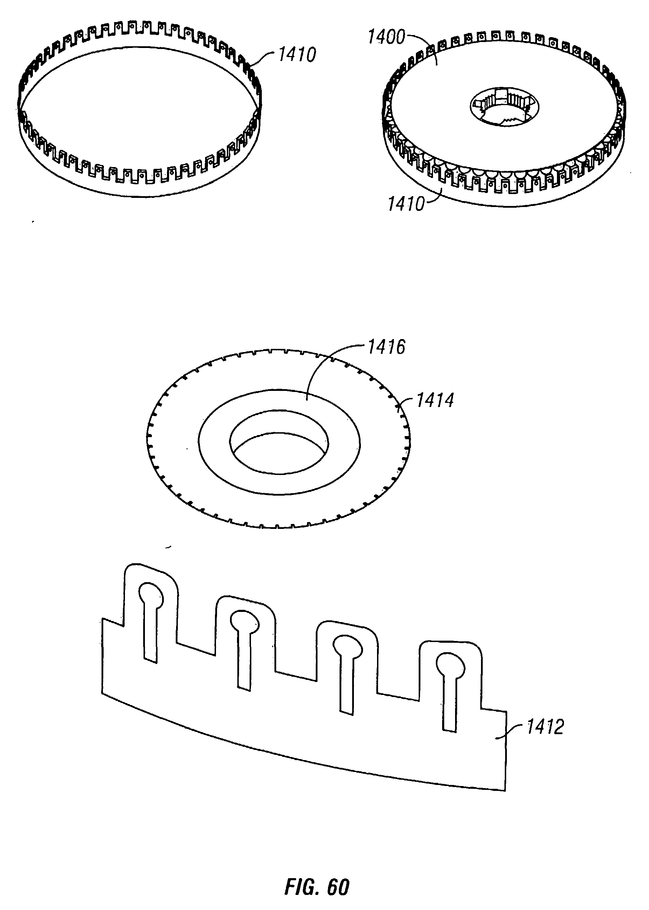

Referring now to Figure 60, yet another embodiment of the present invention will now be described. Dissolvable films have been developed for applications such as cosmetics, oral therapeutic agents, and food products. The unique properties of these materials include a finely tunable dissolution rate, and the ability to alter the surface texture. Additionally, the thickness of the film can be controlled to relatively tight tolerances. These films can function after prolonged exposure to typical environmental conditions, and can be surface treated to withstand higher levels of prolonged humidity.

The electrochemical analyte detecting member used with the present invention may be sensitive to moisture, which can inactivate the sensor. In one embodiment, a hermetic seal is not required for these analyte detecting members, only a hygroscopic seal. This makes the invention of a dissolvable seal a means to simplify the design.

In embodiment, these films are generally starch-based, and the presence of certain enzymes in the blood could break down dissolved starch into glucose. This reaction could be a significant source of signal in the electrochemical detection of glucose. This response can be corrected for, or minimized in the design of the sample capture structure to minimize this as a source of signal error. In other embodiments, the film may be made of non-starch based materials or starches that do not breakdown in time to impact a glucose measurement.

In one embodiment, this film also offers the possibility of a top load sensor that minimizes the required volume. Rather than requiring a discrete, fully formed channel, the dissolution can be tuned so that the electrochemical reaction is triggered on the condition that electrical continuity among the sensor electrodes is detected. The trigger may be timed with the known dissolution properties and sample capture surface designed to achieve a consistent signal response. This would obviate the need for a channel vent, and reduce the sample volume requirements.

Figure 60 shows one embodiment of an assembly with a lancing component 1400, an outer strip 1410 of analyte detecting members.. The fluidic channel is made with a structural adhesive 1412 connected to the orange sensor strip. It is principally topped with a non-dissolvable film. Selected areas defined as the sample capture surface are topped with the dissolvable film. In some embodiments, desiccant 1414 may be provided on a bottom layer 1416.

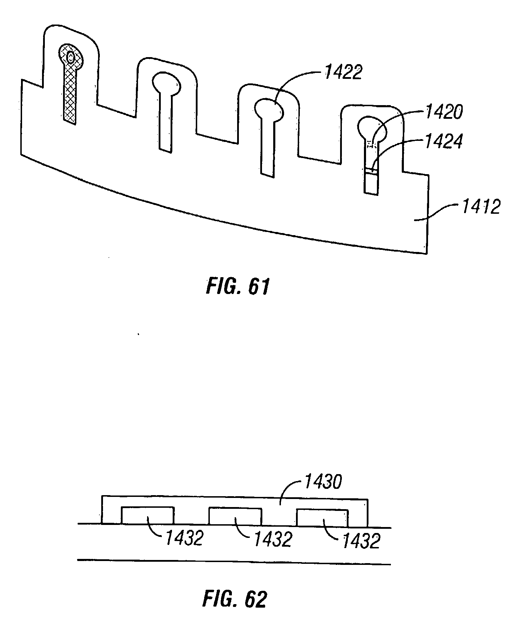

Referring now to the embodiment of Figure 61, this figure is a larger image of the structural adhesive 1412 in Figure 60. It should be understood of course, that the dissolvable film may be adapted for use on a variety if devices and is not limited to use on the structural adhesive 1412. Figure 61 depicts two possible locations of the dissolvable film at location 1420 and 1422. Some embodiments may optionally have second dissolvable film 1424 such shown in Figure 61. Some embodiments may have the dissolvable film 1430 placed directly over the electrodes 1432 as shown in Figure 62. The film may cover all the electrodes or may have individual films covering individual electrodes. The film may have a variety of thicknesses. Some may be as thin as 50 microns. In embodiments using mesh or other transport, in one embodiment, the fluid first dissolves the film and then contacts the mesh or other transport. In some other embodiments, the mesh bring the blood to an area where the film is present and the blood or fluid then dissolves the film to continue on to the electrodes. It typically only takes a few seconds before the film is dissolved. The film may be adapted for use with the electrodes and sample capture devices used with a cartridge as shown in Figure 6.

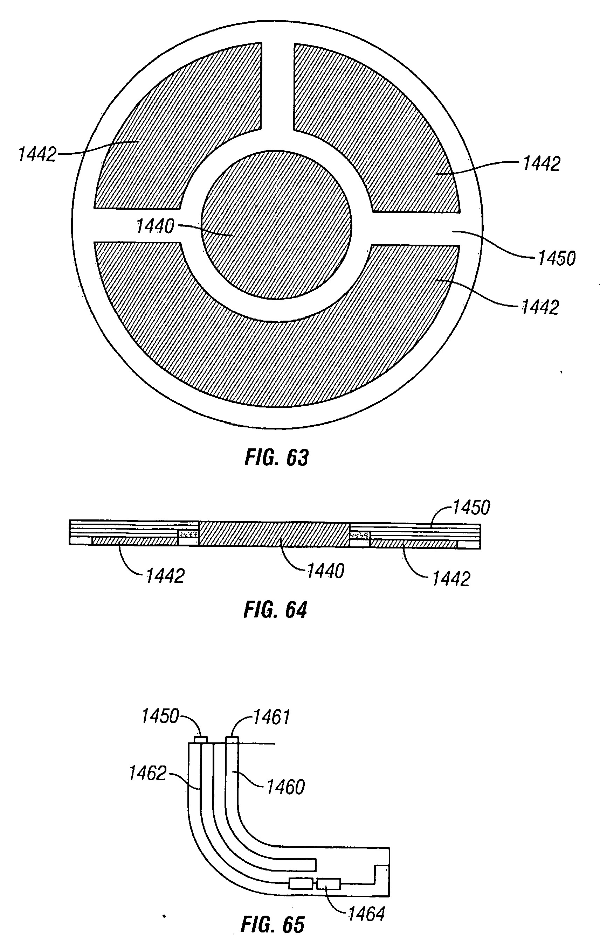

Referring now to the embodiment of Figure 63, a top loading configuration is shown with a lancet aperture 1440 that passes through the analyte detecting member. In this embodiment, the electrodes 1442 are in close proximity to the penetrating member aperture. They are radially arrayed in this configuration. Figure 64 shows the location of the dissolvable film 1450 more clearly. As seen in Figure 65, PSA or other adhesive may be used with the film 1450 to secure it in position. The film 1250 can be of a relatively small footprint, such as but not limited to 3 mm in diameter. In some embodiments, desiccant may be molded in housing or materials near the dissolvable seal. In some embodiments, the vent 1460 may also be covered with a dissolvable seal 1461 and may also dissolve when blood contact the seals. A blood channel 1462 bring blood or fluid to the electrodes 1464.

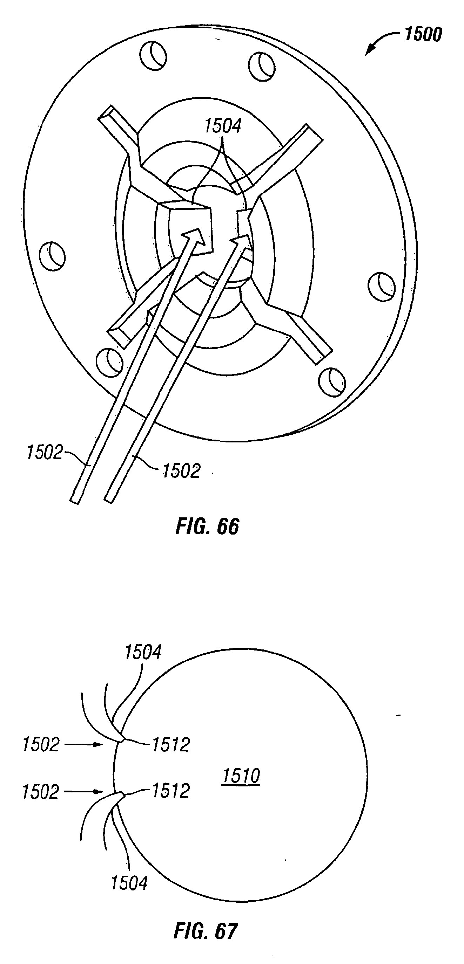

Referring now to Figure 66, yet another embodiment of the present invention will now be described. With any penetrating member actuator assembly, there is a location where the penetrating member interfaces the skin. This interface, or "front end interface" is usually a surface that is flat or contoured to accept a suitable skin lancing site, such as the finger. Within this surface is an opening of various sizes or shapes that the penetrating member passes through when piercing the skin.

To combine the lancing and sensing steps into an integrated, seamless process, a spontaneously derived blood sample on the skin surface is captured with a fluidic system collocated with the penetrating member insult. It is an advantage to have a miniaturized integrated disposable test cartridge that allows for several test units to be placed within close proximity next to each other.

Each wound bleeds differently from one lancing event to the next. The relative location and size of the blood droplet should be well suited to the sample capture surface. In the cases when the droplet is not well controlled, a secondary structure that prevents blood movement across the test cartridge onto an unused unit is desirable to eliminate the risk of cross contamination.

To solve this problem, the present invention in one embodiment, provides a rubberized penetrating member/skin front end interface 1500 that forms a seal when the finger, or other lancing site, depresses the structure. Finger pressure bends the front end interface inwards as indicated by arrows 1502 and the back surface 1504 of the interface mates to the test cartridge 1510 and forms a seal. The seam of this seal forms a perimeter around the active sample capture unit. Some embodiments of the cartridge may have troughs or cutouts 1512 to mate with the back surface 1504. Figure 66 shows one embodiment of the front end. The feature that seals the active analyte detecting member from an unused analyte detecting member.

As seen in Figure 67, in the event that there is too much blood, the blood is confined to the active sample capture unit. Excess blood is captured by the front end interface. The front end interface is removable and cleanable without effecting the rest of the device or the test cartridge. In this manner the lancing site is consistently positioned relative to the penetrating member's travel while allowing the multiple unit cartridge to be freely repositioned when the user is not depressing the interface.

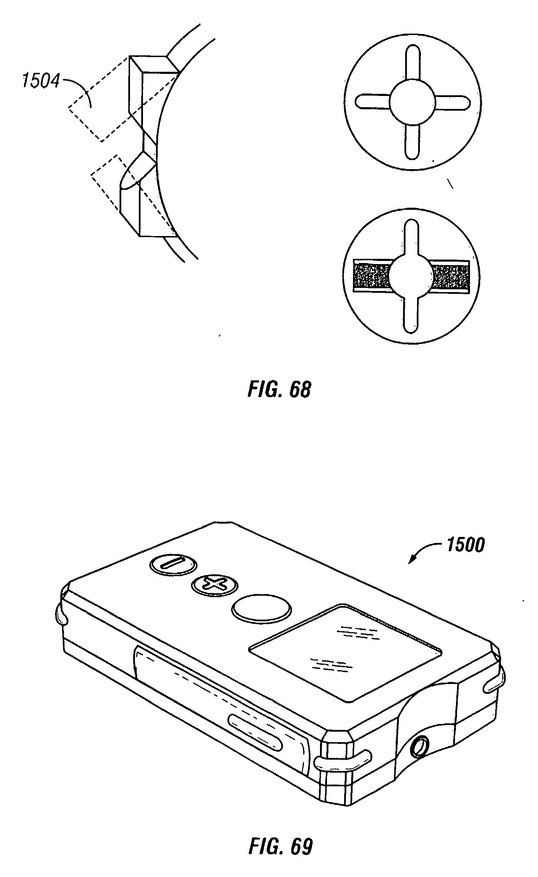

Figure 68 shows that the portions 1504 may engage mating portions shaped to receive the portions identified. This may be trough or valley area that will trap an excess fluid in the area and prevent them from spreading or flowing laterally. An embodiment having a two flaps oriented vertically instead of horizontally may also be used with the present invention. Embodiments having three flaps, four flaps, five flaps, or more which hit a hard stop may also be used with the present invention.

Figure 69 shows one embodiment of the present invention with a interface 1500 mounted on the housing of fluid sampling device.

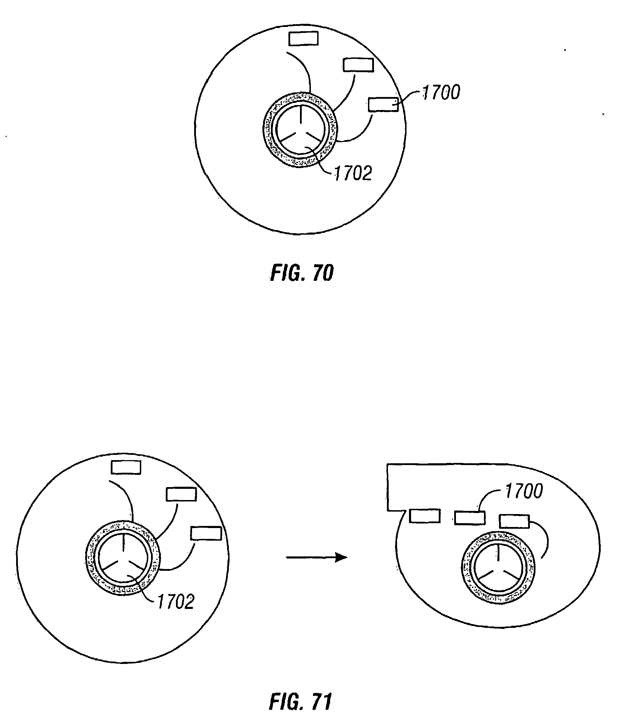

Referring now to Figure 70, analyte detecting members 1700 can be arranged in an annular ring, on the front face of the disposable, there is no lollipop type capture mechanism to bring the blood from the front face down to the analyte detecting member chamber. Rather, the blood is capture directly on the face and transported to the analyte detecting members. This would highlight the aspect that the tip of the penetrating member 1702 is in fluidic contact with the analyte detecting member.

Referring now to Figure 71, in order to further optimize for low volume, the present invention could apply a concentric hydrophillic sample capture and thus an embodiment with a sip-in sample capture is envisaged.

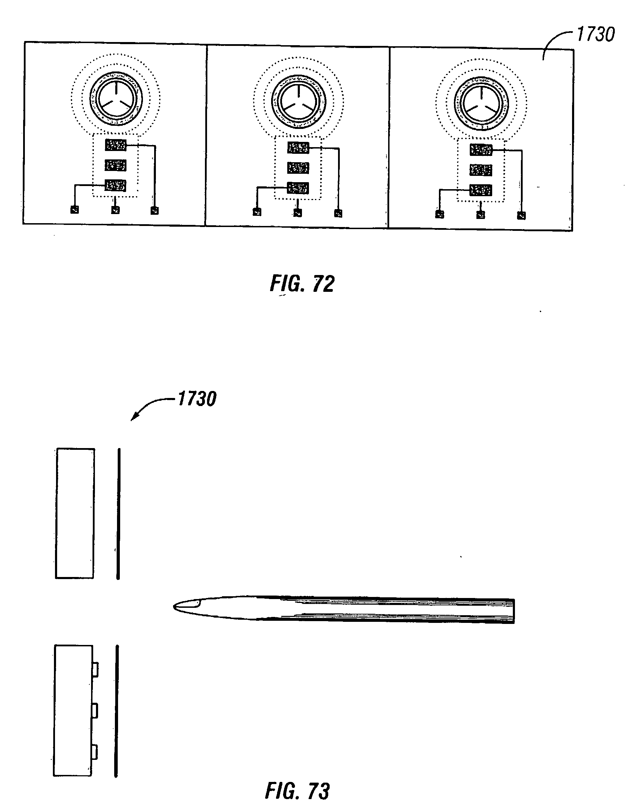

Referring now to Figure 72, the present invention may also be in one embodiment, a film or tape 2730 of analyte detecting members interfaced with the lancing device. The analyte detecting member sample capture aspect could be modified to include the pinwheel sip in structure described above in Figure 71. The analyte detecting member could be moved to the back side of the film so that there would be a dual ring structure, a hydrophilic membrane with sip in integrated into the cover film. The height of the cover film over the analyte detecting member would be 150 mm, the size of the analyte detecting member is 500 mm. The penetrating member could be parked at the sample capture/analyte detecting member interface to further underscore fluidic co-location and enhance sample capture through microfluidic pull due to the facets.

Referring now to Figure 73, analyte detecting members may be on the back of the film, reader on the inside so that there are no through connections required.

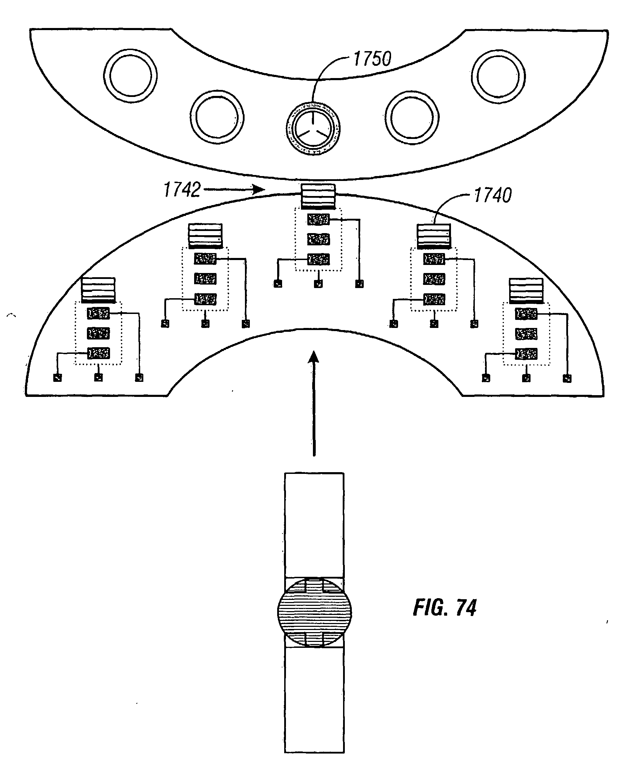

Referring now to Figure 74, an embodiment with independently movable sippers 1740 is shown. A bandolier of sippers 1740 with a lollipop front end could be moved across the sample capture port after sample capture has been achieved. Motion is indicated by arrow 1742. Sample capture by forming a "lens" has been shown in the lab. The sipper 1740 can be moved to the prefilled lens and sample transferred via a mesh tongue. This approach would satisfy the sample capture while displacing the analyte detecting member. In some embodiments, two discs are used, one for the sippers 1740 and one for the blood from the lancing. The discs may rotate together simultaneous or in one embodiment, the blood stays stationary while the sipper 1740 is moved to wipe across the blood 1750. The sequence of the wipe motion may also vary. The sipper 1740 may be constant motion during the lancing event. Others may move after the lancing event occurs. Others may start before the lancing event.

In yet another embodiment, the present invention provides improved pentrating member actuation devices. Shafts are common machine elements. Among many other uses, shafts are used to connect gears and levers to transmit torque and transfer motion within a machine. Shafts are fabricated from flexible materials such as plastics and wrapped wire as in speedometer cable. Shafts are also fabricated from rigid materials such as steel, brass, aluminum etc. Conventional shafts respond to torque by twisting. This twist is proportional to the level of torque applied, and is recovered when the torque is removed (a torsion spring).

In some designs, relative motion between the ends of a shaft is desirable. There are mechanism elements common in the art for achieving relative motion. Examples are; differentials, phase plates, and servo motors.

Embodiments of the present invention relates to a novel shaft construction that allows relative motion of machine elements. A test specimen has been constructed using pultruded carbon fiber material.

In one embodiment, a shaft was made by bonding through injection molding Nylon 6 elements to opposite ends of a 1mm diameter carbon fiber rod. The rod was fabricated by impregnating carbon fibers with a binding matrix, then pulling the wetted bundle of fibers through a die that compressed the fibers into a dense rod and cured the binder. This process is commonly known as pultrusion. In the resulting rod, all of the fibers run parallel to each other along the axis of the shaft.

In testing the bond strength of the Nylon elements, it was observed that the rod behaved as a conventional torsion spring with torsional deflection proportional to the applied torque, and acting to restore the shaft to its original un-deflected shape. Beyond a particular torque level, the shaft began to yield and take a permanent set. Our assumption is that the carbon fibers began breaking loose from the binder and sliding within "tubes" created in the binder. The unique characteristic of this material is that it created a "dead spot" in the torsional response to torque where the restoring force was lost. As the shaft was turned through this dead spot it exhibited a "plastic" behavior in that it could be moved to some deflection with no restoring force. When the shaft was twisted beyond its yield point again, the dead spot was increased. A 720 Degree dead spot was observed.

A pultruded carbon fiber shaft can be used to allow relative rotation of machine elements within a springy "stops" without additional components, saving weight, space and complexity, and improving reliability. An example of such motion can be found in rotating structures such as sensor heads, scanners, or telescopes where support cabling prevents free 360 Degree rotation. A sensor head might be mounted on a carbon fiber shaft and driven through a scan of 540 Degrees with an independent motor driving the sensor head through a gear train. The other end of the shaft would be fixed and would provide "mechanical" spring stops at the 540 Degree limits thus protecting the cabling from damage. In this design, no actual stops are required, and the sensor orientation can easily be provided by the carbon fiber shaft running from an anchor point through a conventional bearing.

The present invention may provide a machine element for: 1) controlled rotation, 2) integrated rotation stops, 3) ease of mounting through conventional bearings, and 4) damped rotation. A pultruded carbon fiber shaft can be used to provide an axis of rotation with integrated rotation stops.

Referring now to Figure 76, another embodiment of the present invention will now be described. In this embodiment, an outer ring 2400 is mounted in an air tight manner against an inner ring 2402 on the cartridge 2404. The air tightness is provided by using a tight fitting plastic joint, adequate enough to allow motion between the rings, but insufficient to allow for atmospheric venting and contamination for analyte detecting members housed between the two mating surfaces of the rings. The bottom may be partially open to allow for electrical connection to contact pads on the analyte detecting members held between the rings.

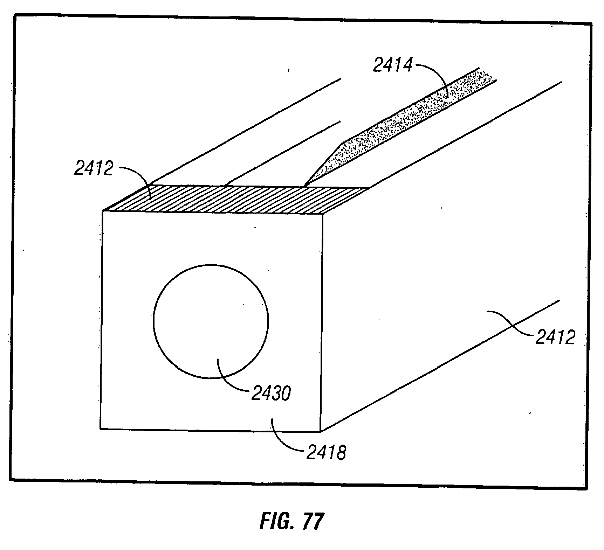

Referring now to Figure 77, a modification to the embodiment shown in Figure 76 will now be described. In Figure 77, there is an outer ring 2410 and this ring 2410 surrounds a cartridge 2412 that contains a plurality of penetrating members 2414. The cartridge 2412 may be a disc that has a flat surface 2416 to engage a flat inner surface 2418 of the ring 2410. The ring 2410 has a slot formed in it to house a plurality of analyte detecting members 2420 which are L-shaped in this embodiment and wherein a portion 2422 is coupled to the underside of cartridge 2412. Thus, the cartridge 2412 and the detecting members 2420 rotate together. Since there is only one opening in ring 2410 (see Figure 76), the relative motion between the ring 2410 and cartridge 2412 allows for only one analyte detecting member to be unsealed at a time. All the other members remain sealed. There is no need of a puncher to open the front end of analyte detecting member since the rotation of the ring will seal and unseal the members as desired.

As seen in Figure 76, there is a common port 2430 with the penetrating member 2414 and the analyte detecting member 2420. Tight fitting plastic material may be used at locations 440 to maintain sterility for other analyte detecting members not in use.

Figure 77 shows a perspective view of one portion of a cartridge 2412 for use with the ring 2410. As seen the front surface 2418 is flat surface for mating with an inner surface of the ring 2410.

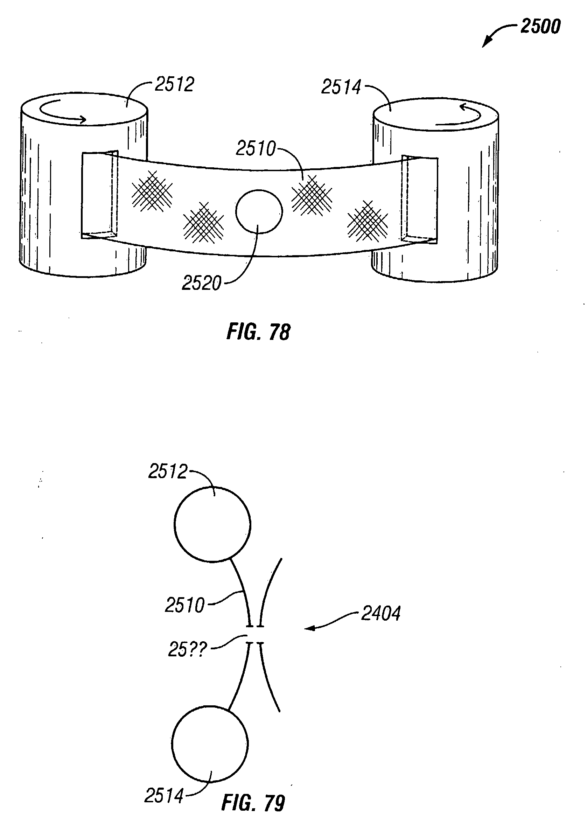

Referring now to Figure 78, yet another embodiment of the present invention will now be described. In this embodiment, an anti-contamination device 2500 is shown. The device 2500 may be used with any of the devices discussed in copending U.S. Patent Application. Ser. No. ____(Attorney Docket No. 38187-2750) filed September 15, 2004 or any embodiments disclosed in U.S. Patent Application. Ser. No. ____(Attorney Docket No. 38187-2608) or U.S. Patent Application. Ser. No.____ (Attorney Docket No. 38187-2662).

In this embodiment, the device 2500 has a film 2510 which is held between a source container 2512 and collection container 2514. In this embodiment, spools may be held in each of the containers 2512 and 2514 for rolling the film therein. The film 2510 may include at least one opening 2520 to allow a user to place tissue therethrough. If the film 2510 is contaminated, the rollers in containers 2512 and 2514 roll to bring a new, clean portion of the film in place to prevent fluid from contacting the cartridge. Some embodiments may bring a new film in position for each test. Some may only do it as desired. There may be enough film for each test on the cartridge 2404. Some may have enough film for 10, 20, 25, 30, 35, 40, 50, 60, 70, 75, 80, 90, 100, or more tests.

Figure 79 shows how one embodiment of the device 2500 may be mounted relative to a cartridge 2404. There device 2500 may be positioned in a horizontal orientation. In other embodiments, the device 2500 may have a vertical orientation relative to the cartridge 2404. The device 2500 may also be placed at other angles relative to the cartridge 2404.

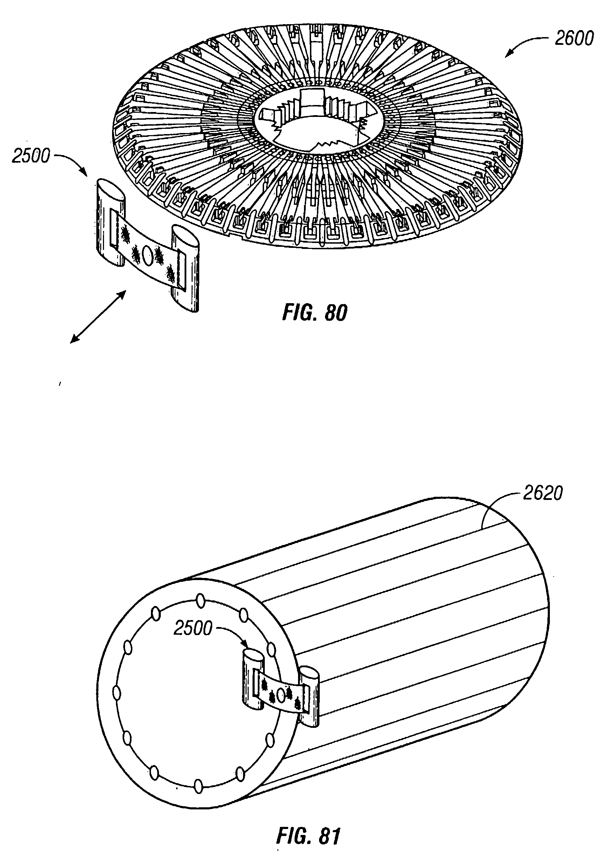

Figure 80 shows another embodiment where the device 2500 is used with cartridge 2600 holding just penetrating members, without sample capture or analyte detecting members.

Figure 81 shows a still further embodiment where the device 2500 is with a cartridge 2620 that is drum shaped and may contain penetrating members. Analyte detecting members or sample capture device may be included with the cartridge 2620. It should be understood that the device 2500 may be used with cartridges of any cross-sectional shape including but not limited to rectangular, circular, hexagonal, polygonal, triangular, oval, ball, or combinations of the above.

The present invention address the problem with smearing leading to a cross contamination of the adjacent unused measuring unit. In particular, if a common port will be used there is a need to have an additional disposable unit to prevent contamination of the common port by blood.

In one embodiment, this disposable consists of 2 canister with an embedded film (film cassette); the film will have 50 holes, one per measuring unit. This disposable will be called as "multi-disposable front-end". It may be located in the device in such a way, that the hole of the film will have a position directly for the aperture of the sample capture structure and thereby a contamination of the common port or the adjacent unused sensor unit(s) will be avoided.

By using this multi-disposable front-end, different patients can use the same fluid sampling system for detecting their glucose level. Therefore, this kind of device can be used in hospitals as well.

Both systems - the lancing device together with the measuring unit and the multi-disposable front end - will form together at least one disposable unit. The movement of this additional disposable unit can be coupled to the movement of cartridge holding the penetrating members.

While the invention has been described and illustrated with reference to certain particular embodiments thereof, those skilled in the art will appreciate that various adaptations, changes, modifications, substitutions, deletions, or additions of procedures and protocols may be made without departing from the invention as defined in claim 1.

The publications discussed or cited herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Expected variations or differences in the results are contemplated in accordance with the objects and practices of the present invention. It is intended, therefore, that the invention be defined by the scope of the claims which follow and that such claims be interpreted as broadly as is reasonable.

## Claims

1. A device comprising:
a cartridge (300) having a plurality of cavities (306); and
a plurality of penetrating members (302) at least partially contained in said cavities (306) of the single cartridge (300) wherein the penetrating members (302) are slidably movable to extend outward (304) from lateral openings (310) on said cartridge (300) to penetrate tissue;
a sterility barrier (434) coupled to said cartridge (300), said sterility barrier (434) covering a plurality of said lateral openings (310), wherein the sterility barrier (434) covering the lateral openings (310) is configured to be moved so that a penetrating member (302) exits the lateral opening without contacting the barrier (434);
a plurality of analyte detecting members (308) coupled to said cartridge (300); and
a plurality of sample capture devices (396), said sample capture devices each having an opening (310) there through to allow a penetrating member (302) to pass through,
**characterised in that**
the device further comprises a dissolvable film adapted for use with the sample capture devices.

2. The device of claim 1 wherein each of said sample capture devices (396) are L shaped.

3. The device of claim 1 or claim 2 wherein said sample capture devices (396) formed on a ribbon or tape structure (364, 2730).

4. The device of any preceding claim wherein said sample capture devices (396) are formed on circular disc (370) and coupled to the cartridge (300).

5. The device of any preceding claim wherein said sample capture devices (396) each includes a wicking member (374).

6. The device of any preceding claim wherein said sample capture devices (396) each includes a hydrophilic membrane (373, 375).

7. The device of claim 5 or claim 6 wherein said sample capture devices each includes a wicking member (374) and a capillary member (380).

8. The device of any preceding claim wherein said sample capture devices (396) each has a lollipop shaped opening on the vertical portion.

9. The device of any preceding claim wherein said sample capture devices (396) are each individually movable to be in an open or a closed position.

10. The device of any preceding claim wherein said sample capture devices (396) are each hinged to a disc (370).

11. The device of any preceding claim wherein said analyte detection members (308) are operable to measure glucose levels in a sample fluid.

12. The device of any preceding claim wherein said penetrating members (302) are lancets (80).

13. The device of any preceding claim comprising a housing (10) and a solenoid penetrating member driver placed in said housing.

14. The device of any preceding claim further comprising provided a plurality of seals (320) on said cartridge to maintain said penetrating members in a sterile condition prior to use.

15. The device of any preceding claim further comprising a housing and an LCD screen provided on said housing to provide information to the user during use. 25957144v.1

## Patentansprüche

1. Vorrichtung, umfassend:
eine Kartusche (300) mit einer Vielzahl von Hohlräumen (306) und eine Vielzahl von Penetrierelementen (302), die mindestens teilweise in den Hohlräumen (306) der einzelnen Kartusche (300) enthalten sind, wobei die Penetrierelemente (302) gleitend beweglich sind, so dass sie sich von seitlichen Öffnungen (310) an der Kartusche (300) nach außen (304) erstrecken, um Gewebe zu penetrieren,
eine an die Kartusche (300) gekoppelte Sterilitätssperre (434), wobei die Sterilitätssperre (434) eine Vielzahl der seitlichen Öffnungen (310) abdeckt, wobei die die seitlichen Öffnungen (310) abdeckende Sterilitätssperre (434) dazu konfiguriert ist, so bewegt zu werden,
dass ein Penetrierelement (302) aus der seitlichen Öffnung austritt, ohne die Sperre (434) zu kontaktieren,
eine Vielzahl von an die Kartusche (300) gekoppelten Analyterfassungselementen (308) und
eine Vielzahl von Probensammelvorrichtungen (396), wobei die Probensammelvorrichtungen jeweils eine Öffnung (310) dort hindurch haben, damit das Penetrierelement (302) hindurchgehen kann,
**dadurch gekennzeichnet, dass**
die Vorrichtung ferner eine lösbare Folie umfasst, die für die Verwendung mit den Probensammelvorrichtungen geeignet ist.

2. Vorrichtung nach Anspruch 1, wobei jede der Probensammelvorrichtungen (396) L-förmig ist.

3. Vorrichtung nach Anspruch 1 oder Anspruch 2, wobei die Probensammelvorrichtungen (396) auf einer Band- oder Streifenstruktur (364, 2730) ausgebildet sind.

4. Vorrichtung nach einem der vorhergehenden Ansprüche, wobei die Probensammelvorrichtungen (396) auf einem kreisförmigen Plättchen (370) ausgebildet und an die Kartusche (300) gekoppelt sind.

5. Vorrichtung nach einem der vorhergehenden Ansprüche, wobei die Probensammelvorrichtungen (396) jeweils ein Element (374) mit Dochtwirkung aufweisen.

6. Vorrichtung nach einem der vorhergehenden Ansprüche, wobei die Probensammelvorrichtungen (396) jeweils eine hydrophile Membran (373, 375) aufweisen.

7. Vorrichtung nach Anspruch 5 oder Anspruch 6, wobei die Probensammelvorrichtungen jeweils ein Element (374) mit Dochtwirkung und ein Kapillarelement (380) aufweisen.

8. Vorrichtung nach einem der vorhergehenden Ansprüche, wobei die Probensammelvorrichtungen (396) jeweils eine lutscherförmige Öffnung am vertikalen Abschnitt haben.

9. Vorrichtung nach einem der vorhergehenden Ansprüche, wobei die Probensammelvorrichtungen (396) jeweils einzeln in eine offene oder eine geschlossene Position beweglich sind.

10. Vorrichtung nach einem der vorhergehenden Ansprüche, wobei die Probensammelvorrichtungen (396) jeweils scharniermäßig mit einem Plättchen (370) verbunden sind.

11. Vorrichtung nach einem der vorhergehenden Ansprüche, wobei die Analyterfassungselemente (308) zum Messen von Blutzuckerwerten in einem Probenfluid betätigt werden können.

12. Vorrichtung nach einem der vorhergehenden Ansprüche, wobei die Penetrierelemente (302) Lanzetten (80) sind.

13. Vorrichtung nach einem der vorhergehenden Ansprüche, umfassend ein Gehäuse (10) und einen Solenoidpenetrierelementtreiber, der im Gehäuse platziert ist.

14. Vorrichtung nach einem der vorhergehenden Ansprüche, ferner umfassend eine Vielzahl von Dichtungen (320) an der Kartusche, um die Penetrierelemente vor der Verwendung in einem sterilen Zustand zu halten.

15. Vorrichtung nach einem der vorhergehenden Ansprüche, ferner umfassend ein Gehäuse und ein am Gehäuse vorgesehenes LCD-Fenster, um dem Benutzer während der Verwendung Informationen zu geben.

## Revendications

1. Dispositif comprenant :
une cartouche (300) comportant une pluralité de cavités (306) ; et
une pluralité d'éléments pénétrants (302) contenus au moins partiellement dans lesdites cavités (306) de la cartouche unique (300), dans lequel les éléments pénétrants (302) sont mobiles par coulissement pour s'étendre vers l'extérieur (304) à partir d'ouvertures latérales (310) sur ladite cartouche (300) afin de pénétrer dans les tissus ;
une barrière anticontamination (434) accouplée à ladite cartouche (300), ladite barrière anticontamination (434) couvrant une pluralité desdites ouvertures latérales (310), dans lequel la barrière anticontamination (434) couvrant les ouvertures latérales (310) est configurée pour se déplacer de façon à ce qu'un élément pénétrant (302) sorte de l'ouverture latérale sans entrer en contact avec la barrière (434) ;
une pluralité d'éléments (308) de détection d'analytes accouplés à ladite cartouche (300) ; et
une pluralité de dispositifs (396) de capture d'échantillons, lesdits dispositifs de capture d'échantillons comportant chacun une ouverture (310) les traversant pour permettre à un élément pénétrant (302) de traverser,
**caractérisé en ce que** le dispositif comprend en outre un film soluble apte à être utilisé avec les dispositifs de capture d'échantillons.

2. Dispositif selon la revendication 1, dans lequel chacun desdits dispositifs (396) de capture d'échantillons est en forme de L.

3. Dispositif selon la revendication 1 ou 2, dans lequel lesdits dispositifs (396) de capture d'échantillons sont formés sur une structure (364, 2730) en ruban ou bande.

4. Dispositif selon l'une quelconque des revendications précédentes, dans lequel dans lequel lesdits dispositifs (396) de capture d'échantillons sont formés sur un disque circulaire (370) et accouplés à la cartouche (300).

5. Dispositif selon l'une quelconque des revendications précédentes, dans lequel dans lequel lesdits dispositifs (396) de capture d'échantillons comprennent chacun un élément (374) à effet de mèche.

6. Dispositif selon l'une quelconque des revendications précédentes, dans lequel dans lequel lesdits dispositifs (396) de capture d'échantillons comprennent chacun une membrane hydrophile (373, 375).

7. Dispositif selon la revendication 5 ou 6, dans lequel dans lequel lesdits dispositifs de capture d'échantillons comprennent chacun un élément (374) à effet de mèche et un élément capillaire (380).

8. Dispositif selon l'une quelconque des revendications précédentes, dans lequel dans lequel lesdits dispositifs (396) de capture d'échantillons comportent chacun une ouverture en forme de sucette sur la partie verticale.

9. Dispositif selon l'une quelconque des revendications précédentes, dans lequel dans lequel lesdits dispositifs (396) de capture d'échantillons sont chacun mobiles individuellement pour être dans une position ouverte ou une position fermée.

10. Dispositif selon l'une quelconque des revendications précédentes, dans lequel dans lequel lesdits dispositifs (396) de capture d'échantillons sont chacun articulés sur un disque (370).

11. Dispositif selon l'une quelconque des revendications précédentes, dans lequel lesdits éléments (308) de détection d'analyte servent à mesurer les niveaux de glucose dans un fluide échantillon.

12. Dispositif selon l'une quelconque des revendications précédentes, dans lequel dans lequel lesdits éléments pénétrants (302) sont des lancettes (80).

13. Dispositif selon l'une quelconque des revendications précédentes, comprenant un boîtier (10) et un entraînement par solénoïde d'élément pénétrant placé dans ledit boîtier.

14. Dispositif selon l'une quelconque des revendications précédentes, comprenant en outre la mise en place d'une pluralité de joints d'étanchéité (320) sur ladite cartouche pour maintenir lesdits éléments pénétrants dans un état stérile avant utilisation.

15. Dispositif selon l'une quelconque des revendications précédentes, comprenant en outre un boîtier et un écran à cristaux liquides disposé sur ledit boîtier pour fournir des informations à l'utilisateur pendant l'utilisation.
